# EUROPEAN PATENT SPECIFICATION

(11) **EP 3 525 674 B1**
(45) Date of publication and mention of the grant of the patent: **08.12.2021**
(21) Application number: 17860347.8
(22) Date of filing: 15.10.2017
(51) Int. Cl.: A61B 5/1455, A61B 5/02, A61B 5/024

(54) **METHOD AND APPARATUS FOR OPTICALLY MEASURING BLOOD PRESSURE**
VERFAHREN UND VORRICHTUNG ZUR OPTISCHEN MESSUNG DES BLUTDRUCKS
PROCÉDÉ ET APPAREIL DE MESURE OPTIQUE DE LA PRESSION ARTÉRIELLE

(30) Priority: 12.10.2016 US 201662407429 P; 07.06.2017 WO PCT/IB2017/053339
(43) Date of publication of application: 21.08.2019
(73) Proprietor: Elfi-Tech Ltd., 7670502 Rehovot (IL)
(72) Inventor: FINE, Ilya, 7630242 Rehovot (IL); KAMINSKY, Alexander, Tbilisi 0186 (GE)
(74) Representative: Harrison IP Limited
(86) International application number: PCT/IL2017/051139
(87) International publication number: WO 2018/069931

(56) References cited:
- EP-A1- 0 537 383
- US-A- 4 726 382
- US-A1- 2011 295 133
- US-A1- 2012 130 215
- US-B2- 6 801 798
- US-B2- 7 341 560

## Description

### BACKGROUND

### Blood Pressure

Blood pressure is defined as the pressure that is exerted by the blood upon the walls of the blood vessels and especially arteries and that varies with the muscular efficiency of the heart, the blood volume and viscosity, the age and health of the individual, and the state of the vascular wall

Blood pressure is recorded as two numbers, such as 120/80. The larger number indicates the pressure in the arteries as the heart pumps out blood during each beat. This is called the systolic blood pressure. The lower number indicates the pressure as the heart relaxes before the next beat. This is called the diastolic blood pressure.

Elevated blood pressure above its 'normal' range is a significant cause of death and disability in the world. Accurate blood pressure measurement is therefore vital in the prevention and treatment of blood-pressure-related diseases. Additionally, in very ill patients, accurate measurement of blood pressure is essential for monitoring cardiovascular homeostasis.

The most accurate non-invasive method of blood pressure measurement is called auscultatory or Korotkoff method. It is based on the observation of the repetitive sounds generated by the blood flow. As the cuff pressure decreases gradually during deflation, the Korotkoff sound changes in intensity and quality, and five different stages can be distinguished. The blood pressure measurement based on Korotkoff sounds using mercury sphygmomanometer currently is regarded as the gold standard method for indirect measurement of blood pressure.

The *Wikipedia* article about "Sphygmomanometer" states the following:
With a manual instrument, listening with a stethoscope to the brachial artery at the elbow, the examiner slowly releases the pressure in the cuff. As the pressure in the cuffs falls, a "whooshing" or pounding sound is heard (see Korotkoff sounds) when blood flow first starts again in the artery. The pressure at which this sound began is noted and recorded as the systolic blood pressure. The cuff pressure is further released until the sound can no longer be heard. This is recorded as the diastolic blood pressure. In noisy environments where auscultation is impossible (such as the scenes often encountered in emergency medicine), systolic blood pressure alone may be read by releasing the pressure until a radial pulse is palpated (felt). In veterinary medicine, auscultation is rarely of use, and palpation or visualization of pulse distal to the sphygmomanometer is used to detect systolic pressure.

The major disadvantage of this method that this type of measurement needs a physician or specially trained care-giver.

In contrast, home self-monitoring of the blood pressure is preferably performed automatically, obviating the need for requiring a trained care-giver. Typically, commercially available home self- devices employed an oscillometric method of the blood pressure.

Oscillometric measurement devices use an electronic pressure sensor with a numerical readout of blood pressure. In most cases the cuff is inflated and released by an electrically operated pump and valve, which may be fitted on the wrist (elevated to heart height), although the upper arm is preferred. Initially, the cuff is inflated to a pressure in excess of the systolic arterial pressure, and then the pressure reduces to below diastolic pressure. Once the blood flow is present, but restricted, the cuff pressure will vary periodically in synchrony with the cyclic expansion and contraction of the brachial artery. The values of systolic and diastolic pressure are computed from the raw data, using a specially adjusted algorithm. Most of the oscillometric algorithms rely on empirical coefficients for systolic and diastolic pressure evaluation that may differ in various patient populations. This makes this technique less accurate and reliable than auscultatory method.

### Optical Blood Motion Sensors

For the present disclosure, a 'blood motion' sensor measures motion of blood or any component (e.g. blood plasma, red blood cells (RBCs) suspended in blood plasma) thereof through blood vessels (e.g. skin capillaries).

Types of blood motions sensors include blood flow sensors (e.g. laser-Doppler sensors, or DLS (dynamic-light-scattering) sensors), pulse sensors (PPG) techniques or laser-Doppler-based pulse wave sensors or DLS-based pulse wave sensors).

One salient feature of blood motion sensors, especially PPG based sensors, is that it is desirable for direct contact between a light source of the sensor and the subject's skin - this allows for the most accurate measurement of blood motion, and in the case of PPG is an enabling required - i.e. without such direct contact, meaningful measurement of blood flow may be impossible.

### DLS Sensors

PPG and laser-Doppler sensors have been known in the art for many decades. In contrast, DLS sensors (used interchangeably with 'DLS devices') were disclosed much more recently. Thus, in recent years, it has been disclosed to employ dynamic light scattering techniques (DLS) to measure pulse, blood pressure, blood plasma viscosity, and other hemodynamic parameters - see WO 2008/053474, WO/2011/013132, WO/2012/064326, and WO/2016/185244.

WO 2008/053474 and WO/2012/064326, disclose detecting and analyzing a fluctuation-dependent speckle pattern by dynamic light scattering techniques. It is disclosed that the blood pressure may be computed according to the results of the analysis. The DLS method is based on the effect of coherent light scattering on moving particles. In the case of the measurement of blood pressure the Red Blood Cells (RBC's) moving in the arterial and capillary blood path are responsible for the measured DLS-related signal. In addition, WO/2012/064326 discloses an apparatus comprising first and second photodetectors configured to respectively generate first and second analog signals from light incident thereon. Analog circuitry generates a difference analog signal from the first and second analog signal and this difference analog signal is analyzed, for example, using DLS techniques and/or to detect blood pressure.

When optically probing pulsatile blood, DLS devices work as follows: (i) a portion of the subject's skin or tissue is illuminated by source of coherent light (e.g. a VCSEL (vertical cavity surface emitting laser) or a diode laser) to scatter partially or entirely coherent light off of the subject's moving red blood cells (RBCs) to induce a scattered-light time-dependent and shear-rate dependent optical response; (ii) the scattered light from the illuminated skin or tissue is received by a photodetector(s) to generate an electrical signal descriptive of the induced scattered-light time-dependent optical response; (iii) the scattered-light-optical-response-descriptive electrical signal or a derived-signal thereof is processed (e.g. by any combination of analog circuitry, digital circuitry such as a digital computer, firmware and/or software) to compute therefrom one or more blood-shear-rate-descriptive (BSRD) signal(s). This processing of the scattered-light-optical-response-descriptive electrical signal or a derived-signal thereof may be performed by computing an autocorrelation of the scattered-light-optical-response-descriptive electrical signal or by computing a power spectrum thereof.

Fig. 11B of WO 2008/053474 illustrates an example of a BSRD signal(s) that describes a pulsatile pressure wave - i.e. a pulsatile BSRD. As discussed in WO 2008/053474, the pulsatile BSRD may be analyzed, for example, to compute a subject's pulse rate.

In contrast to PPG devices, which may only be used to measure systolic blood pressure, WO 2008/053474 teaches that DLS devices may be used to measure systolic or diastolic blood pressure.

### Optical Blood Pressure Sensors

US 20120179011 discloses a body-worn system that continuously measures pulse oximetry and blood pressure, along with motion, posture, and activity level, from an ambulatory patient. Fig. 1 (PRIOR ART) roughly corresponds to Fig. 23A of US 20120179011 and shows that the system of US 20120179011 includes: (i) an inflatable cuff disposed on the subject's upper arm and (ii) a PPG device disposed 'downstream' to the inflatable cuff on the subject's finger.

Thus, in US 20120179011 there is a longitudinal displacement (e.g. on the order of magnitude of 30-70 centimeters) between (i) the 'blood occluding location' where a pressure-applying surface (i.e. of the cuff) applies a blood-occluding pressure on the subject to occlude blood flow at downstream locations and (ii) the blood-motion measurement location where blood motion (i.e. in the example of US 20120179011 this is pulse) is optically measured. As shown in Fig. 1, this measurement location is significantly distal (i.e. towards the subject's fingertip) to the pressure-applying surface.

US 20120179011 is not the only disclosure of optical blood pressure sensors - other disclosures of optical blood pressure sensors appear in the literature (e.g. see WO 2008/053474).

There is an ongoing need for accurate and easy-to-use blood pressure sensors, suitable for home use. To date, market adaption of optical pressure sensing technology has been minimal at best.

Additional prior art documents include US patent 6801798, pre-grant publication US 2012/130215, pre-grant publication US 2011/295133, US patent 7341560 and pre-grant publication EP 0537383 (publication of EP application 91202676.2).

### SUMMARY OF EMBODIMENTS

The invention is best summarised by the independent claims. Further advantageous embodiments are found in the dependent claims.

### BRIEF DESCRIPTION OF THE DRAWINGS

Some embodiments of the printing system are described herein with reference to the accompanying drawings. The description, together with the figures, makes apparent to a person having ordinary skill in the art how the teachings of the disclosure may be practiced, by way of non-limiting examples. The figures are for the purpose of illustrative discussion and no attempt is made to show structural details of an embodiment in more detail than is necessary for a fundamental understanding of the disclosure. For the sake of clarity and simplicity, some objects depicted in the figures are not to scale.
Fig. 1 (PRIOR ART) illustrates: (i) an inflatable cuff disposed on the subject's upper arm and (ii) a PPG device disposed 'downstream' to the inflatable cuff on the subject's finger.
Figs. 2A-2C, 3A-3B, 4A-4H, 5A-5B, 6, 7A-7C, 8A-8B, 9A-9F, 11A-11D, 12, 13A-13D, 15A-15C, 17A-17D, 22A-22D, 23A-23B illustrate optical blood-pressure sensors or components thereof;
Figs. 10A-10B and 16 are flow charts of methods for optically measuring blood pressure.
Figs. 14A-14B illustrate a blood motion signal as a function of time.
Figs. 18-20 present examples related to a theoretical discussion about measuring systolic and/or diastolic blood pressure, based on the measurement of peripheral blood flow

### DESCRIPTION OF EMBODIMENTS OF THE INVENTION

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. Throughout the drawings, like-referenced characters are generally used to designate like elements.

### DEFINITIONS

For the present disclosure, the term 'optically transparent' refers to light transparent for at least a portion (i.e. at least one wavelength) of the visible and/or infrared (IR) spectra (e.g. near-IR (NIR) spectra up to 1200 nm or at up to 1100 nm or up to 1000 nm).

For the present disclosure, the term 'optically transparent' means transparent to at least a one wavelength of light in the visible and/or infrared (IR) spectrum. In some embodiments, 'optically transparent' refers to transparent to at least one wavelength in the near-infrared (NIR) spectrum.

A 'flexible' object (e.g. barrier portion or inner ring portion) has sufficient flexibility for its intended purpose - i.e. to deform in response to inflation of an inflatable cushion or chamber that is mechanically coupled to the 'flexible' object so as to transfer force from the interior of the inflatable cushion or chamber to a third object (i.e. biological tissue). In some embodiments, any flexible object may be defined by a Shore hardness (e.g. of the inner 104 ring surface) of at most 30 or at most 25 or at most 20, and further optionally, the Shore hardness is at least 10 or at least 15.

For the present disclosure, a 'portion' of an object refers to 'at least a portion.' A 'section' of an object refers to 'at least a section.' Portion and section may be used interchangeably.

For the present disclosure, when an object (e.g. a rigid restrictor or a rigid section of an outer ring) has an 'optically transparent region' the 'optically transparent region' may be any combination of (i) 'optically transparent' material of the object and/or (ii) empty space (e.g. a void or recess or window) within (and defined by) the object - e.g. a canal or window through which visible and/or IR/or NIR light passes.

For the present disclosure, when a cushion or chamber is 'inflated' it is 'at least partially inflated.' In any embodiment, this may be 'at least partially inflated to at least over- systolic pressure.'

For the present disclosure, 'electrical circuitry' or 'electronic circuitry' (or any other circuitry' such as 'blood pressure circuitry' or 'control circuitry' or pump control circuitry') may include any combination of analog and/or digital circuitry and/or software/computer readable code module and/or firmware and/or hardware element(s) including but not limited to a CPU, volatile or non-volatile memory, field programmable logic array (FPLA) element(s), hard-wired logic element(s), field programmable gate array (FPGA) element(s), and application-specific integrated circuit (ASIC) element(s). Any instruction set architecture may be used including but not limited to reduced instruction set computer (RISC) architecture and/or complex instruction set computer (CISC) architecture.

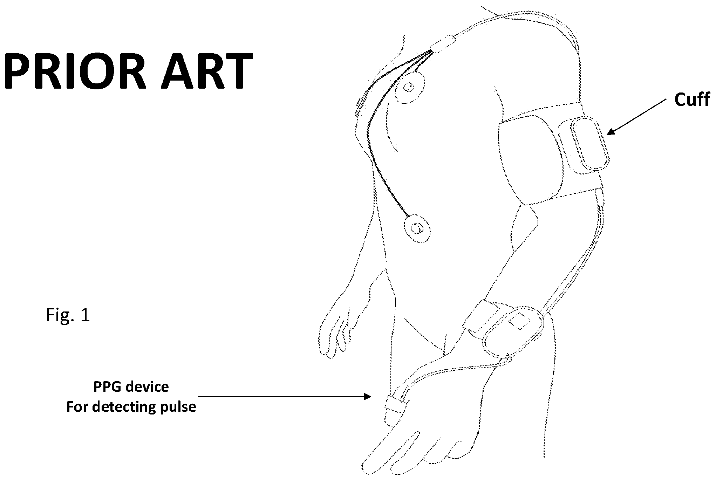

Non-Contact configuration -Some embodiments of the present invention relate to a system and method of optically measuring systolic and/or diastolic blood pressure based upon optically measuring a blood motion signal (e.g. a pulsatile signal) at a location where pressure is applied to biological tissue, rather than at a 'downstream' location as illustrated in Fig. 1. The blood motion is electronically correlated with a magnitude of pressure applied on the biological tissue.

Instead of requiring contact between a laser of an optical blood motion sensor and the biological tissue (as would be necessary in pulse oximeters), it is possible to operate the optical blood motion sensor in a non-contact configuration.' In this non-contact configuration, *en route* to biological tissue, laser light must first traverse at least one of (e.g. any combination of) a flexible and optically transparent (FOT) barrier section of a pressure-applying cushion and/or an interior of the cushion and/or an optically-transparent region of a rigid restrictor (e.g. the laser light must traverse all three). In this manner the blood motion sensor is disposed in a non-contact configuration' and measure blood flow while in the non-contact configuration.

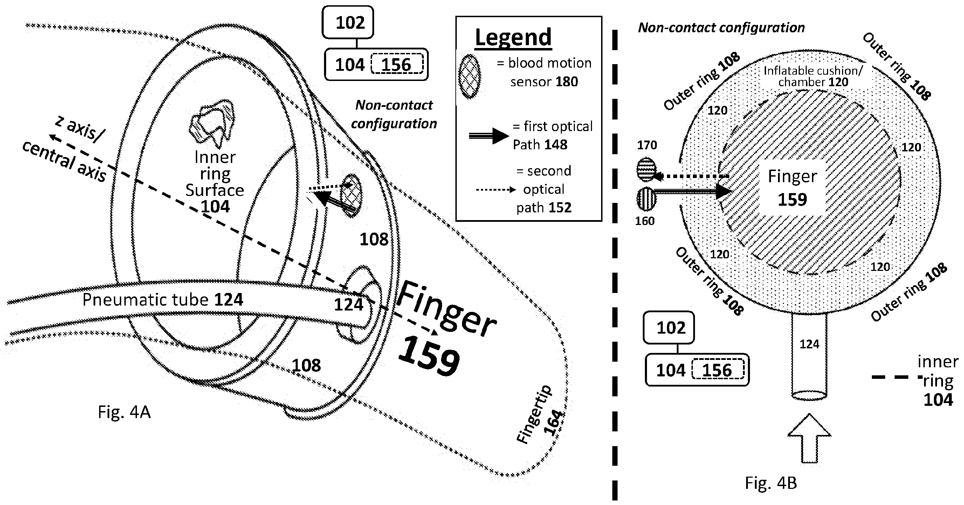

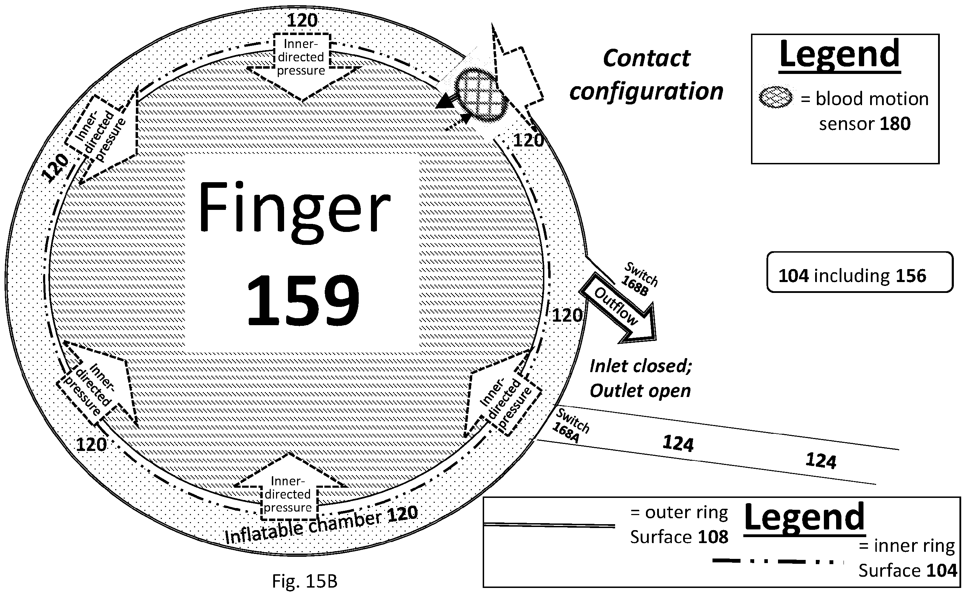

Examples of blood flow sensor operating while in the non-contact configuration' are shown in Figs. 4A-4H, 5A-5B, 6, 7A-7C, 8A-8B, 9A-9F, 11A-11D, 12, 13A, 13C-13D, 15A, 22A-22D, 23A-23C all of which are discussed in greater detail below. In contrast, in the example of Fig. 15B blood motion sensor **180** is in direct contact with biological tissue (i.e. finger **159**) (in a 'contact' configuration) - thus, the chance of mechanical interference from blood motion sensor **180** is greater in the example of Fig. 15B.

Rigid Restrictor -- A presence of the rigid restrictor serves to reduce the amount of time required to inflate the cushion and/or serves to evenly distribute applied pressure around a circumference of the subject's finger of toe. One example of a 'rigid restrictor' is rigid outer ring **108** of an annular-shaped ring assembly (see, for example, Fig. 4B, discussed below). When the restrictor is circular shapes like outer ring **108**, this prevents pressurized gas (or liquid) within chamber **120** from occupied space outside of outer ring **108**, thus 'restricting' a range of motion of the pressurized gas (or liquid).

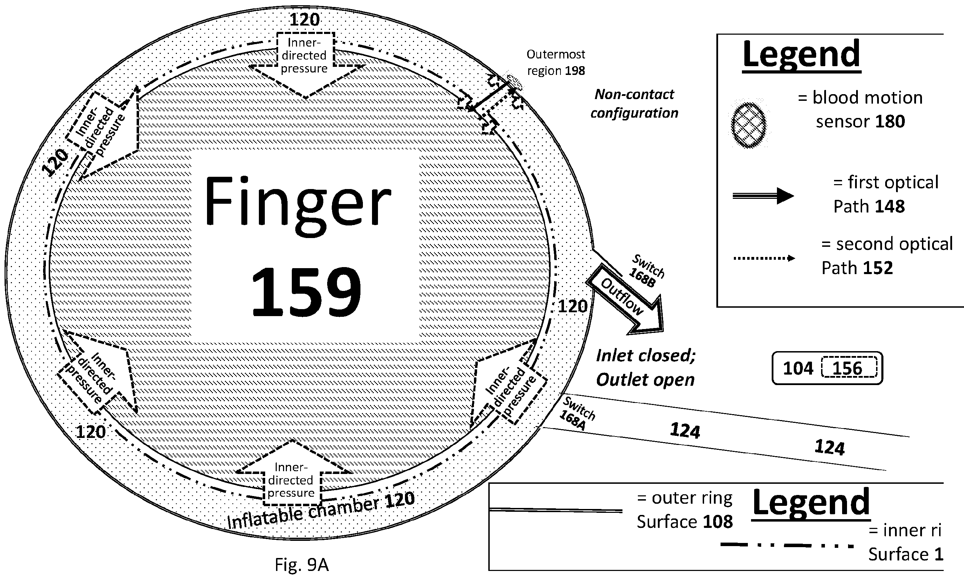

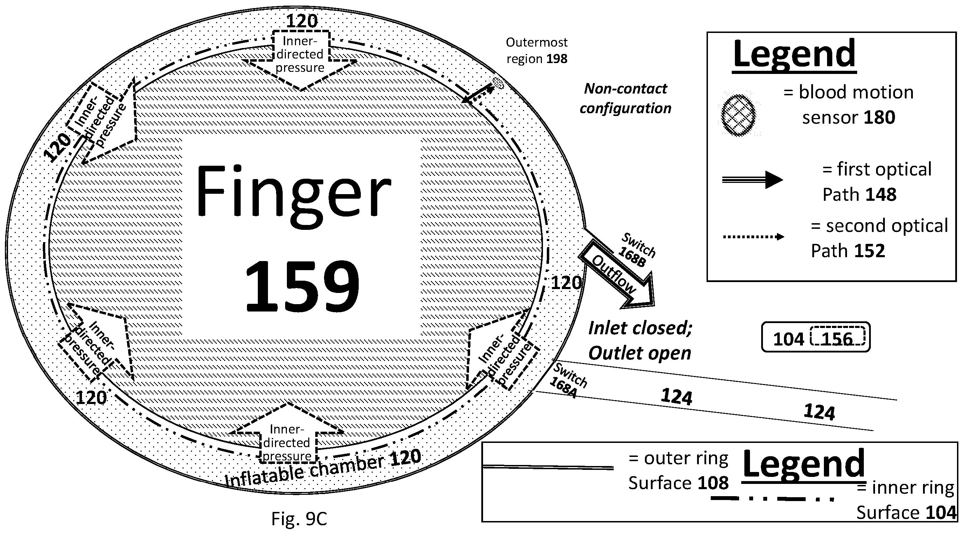

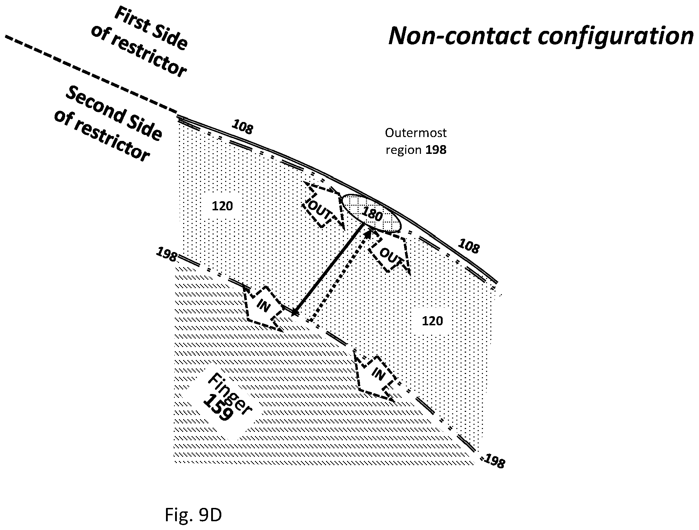

As will be discussed below, in the examples of Figs. 4A-4D, 5A-5B, 6, 7A-7C, 8A-8B, 9A-9B, 9E-9Fm 11A-11B, 13A, 13C-13D, 15A blood flow sensor **180** is both (i) in a non-contact configuration and (ii) in a configuration where the blood floor sensor **180** and the biological tissue **159** where blood pressure is measured are disposed on opposite sides of the rigid restrictor (e.g. at least a portion of outer shell **108).** In contrast, in the example of Figs. 9C-9D, blood floor sensor **180** is also disposed in a non-contact configuration' - however, in the example of Figs. 9C-9D blood floor sensor **180** and the biological tissue **159** are disposed on the same side of rigid restrictor **108.** Without limiting the scope of the invention, it is believed that the configuration of Figs. 9C-9D may be preferable to situations of 'contact configuration' (i.e. less mechanical interference) but less preferable to situations like those of Figs. 9A-9B where in addition to non-contact configuration, the sensor **180** and the tissue **159** are on 'opposite rides' of the rigid restrictor for even less mechanical interference.

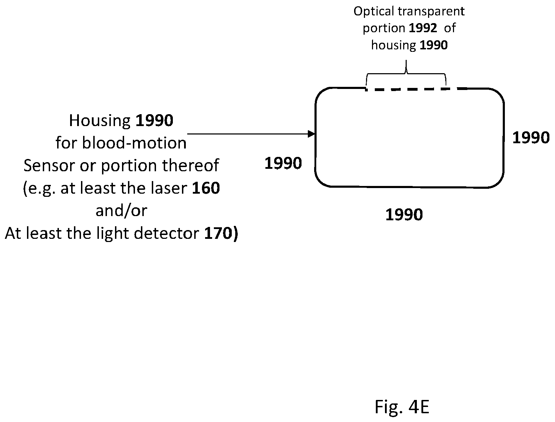

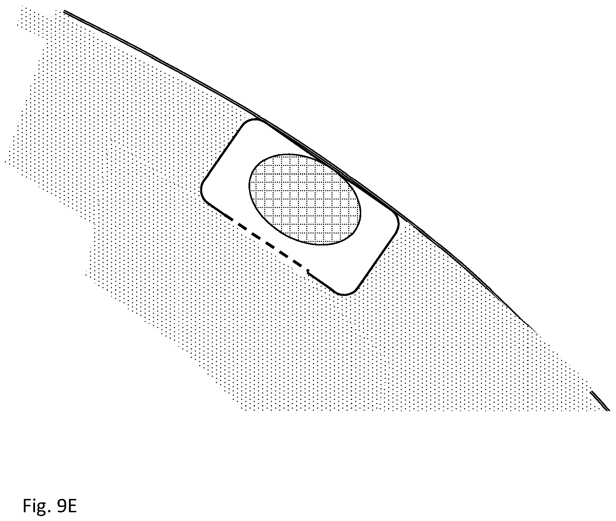

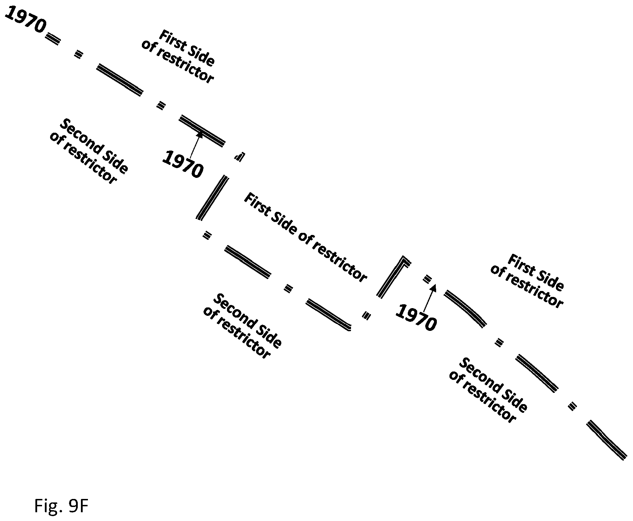

Fig. 9E shows an alternative example relating to the housing 1990 discussed above with reference to Figs. 4E-4H. Figs. 9F shows first and second sides of a rigid restrictor surface 1990 according to the example of Fig. 9E. In the examples of Fig. 9E-9F, the portion of the ring is not the entire restrictor - instead, the rigid restrictor is comprised of the combination of (i) at least a portion of substrate (e.g. at least a portion of the ring) to which the housing **1990** is mounted and (ii) at least a portion of housing **1990** (e.g. having **1992** which may be optically transparent or which may be a void).

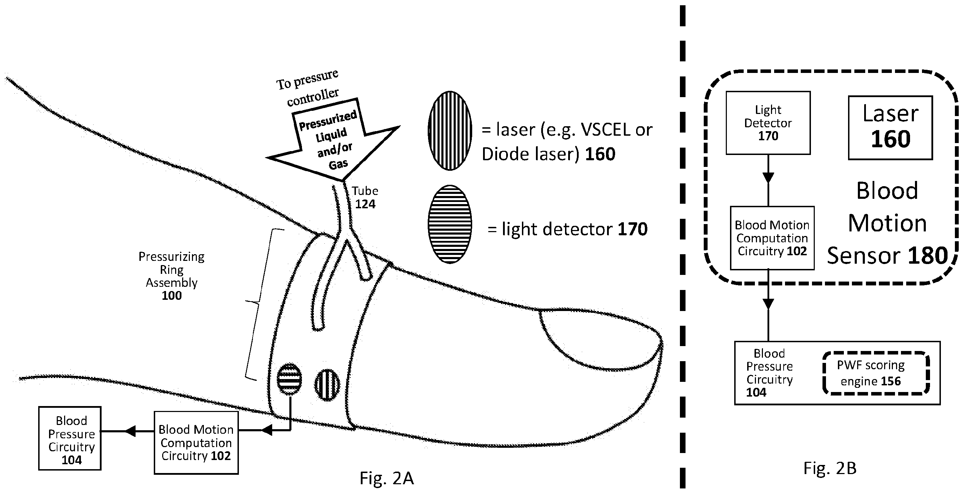

### A Discussion of Figs. 2A-2C, 3A-3B, 4A-4H, 5A-5B, 6, 7A-7C, 9A-9D, 13A, 13C-13D, 15A-15C

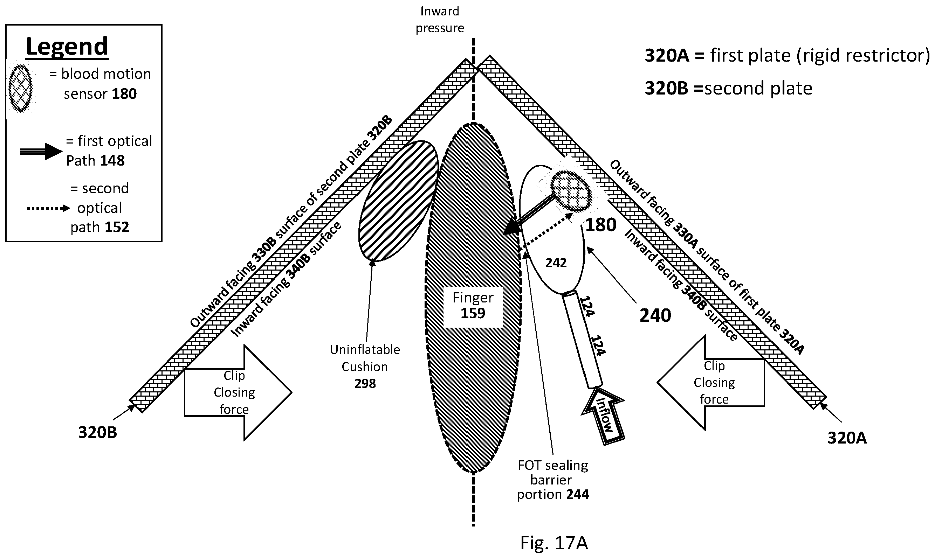

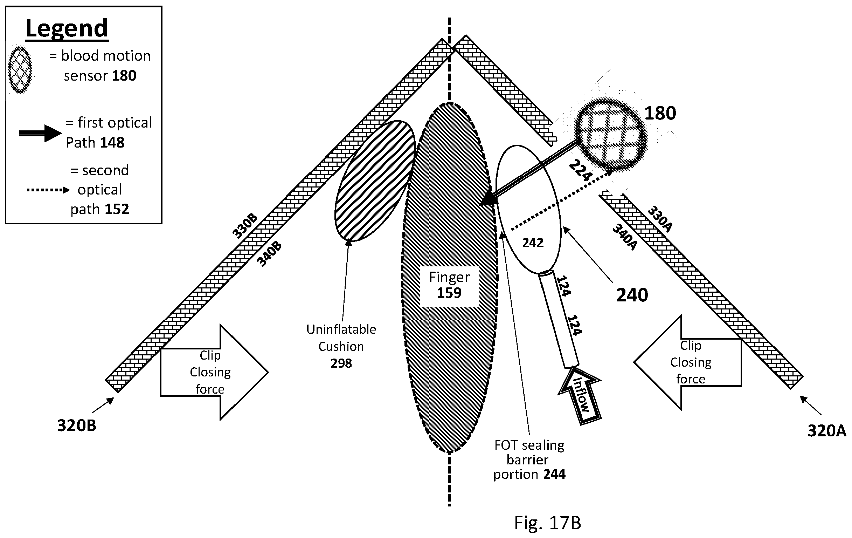

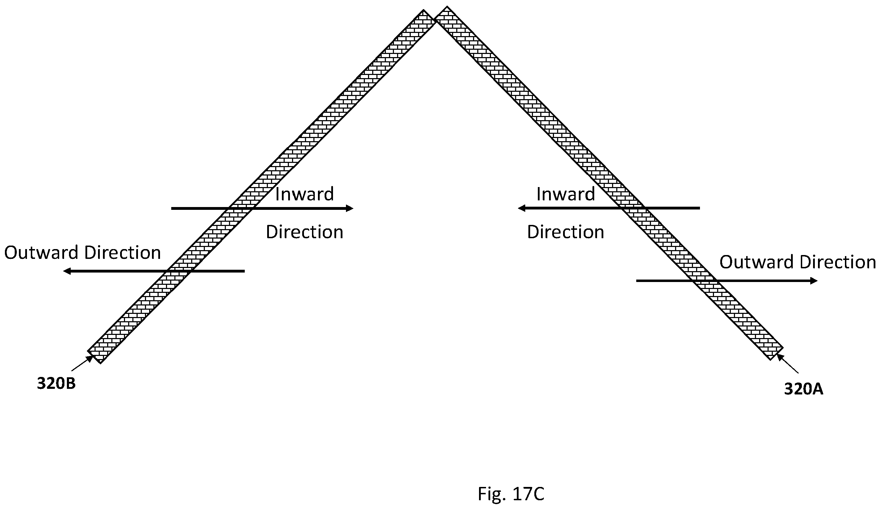

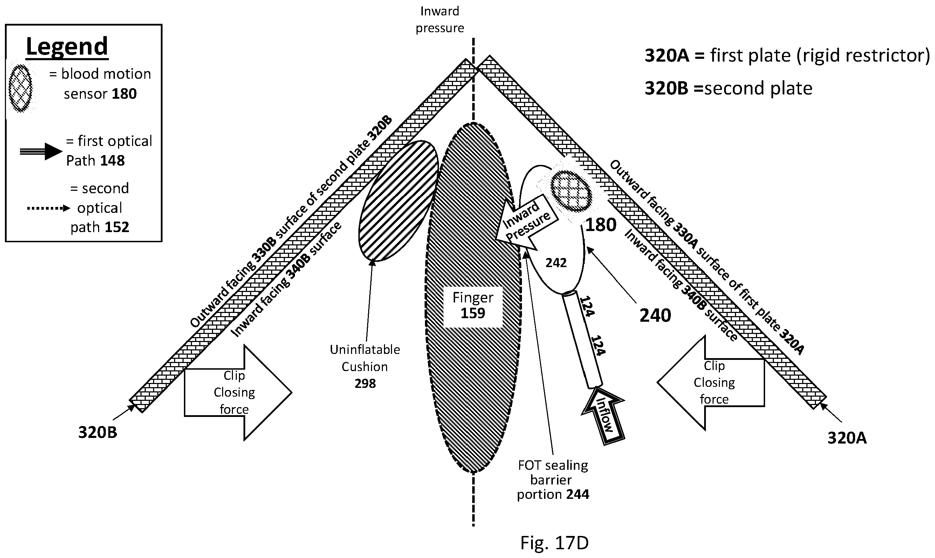

Figs. 2A-2C, 3A-3B, 4A-4D, 5A-5B, 6, 7A-7C, 9A-9D, 13A, 13C-13D, 15A-15C relate to apparatus for measuring systolic and/or diastolic blood pressure comprising a ring assembly. In contrast, the apparatus for measuring systolic and/or diastolic blood pressure illustrated in Fig. 17 has a clip form factor.

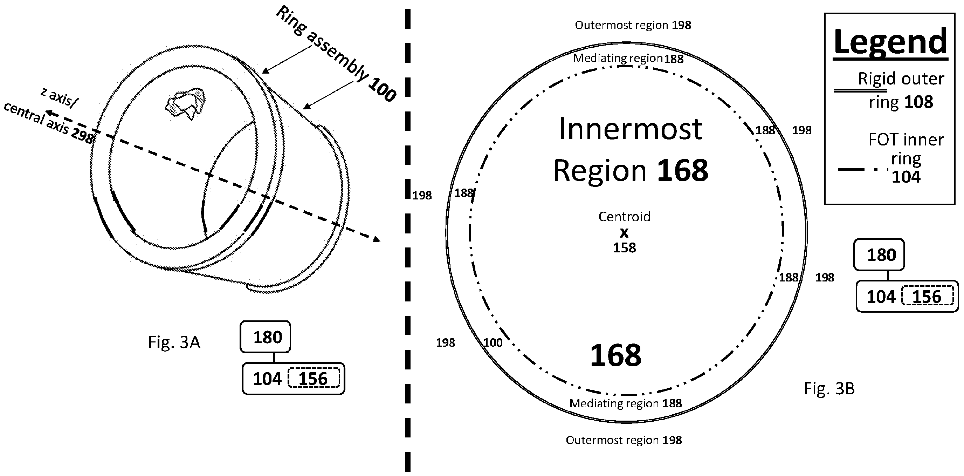

Figs. 3A-3B, 4A-4D, 5A-5B, 6, 7A-7C, 9A-9D, 13A, 13C-13D, 15A-15C are other views of the ring assembly or portions thereof.

As shown in Fig. 2A, laser **160** of optical blood motion sensor **180** illuminates skin of the user's finger so that light reflected by the skin of the illuminated finger is received by light detector **170.** In the particular example of Fig. 2A, both laser **160** and light detector **170** are facing inwards (i.e. towards the finger in this case) and are mounted to an outward-facing surface of the ring assembly. The ring assembly includes an inflatable cushion (not explicitly labelled and/or shown in Fig. 2A) having a barrier for providing a gas-seal between an interior of the cushion **120** and locations outside of the cushion. The barrier has a portion (not shown in Fig. 2A) that is flexible and optically-transparent (FOT) which applies inwardly pressure (e.g. at least systolic pressure) upon the finger of the subject.

Pressurized fluid (e.g. pressurized gas or pressurized liquid) is forced through tube **124** to inflate the cushion and to apply pressure (e.g. inwardly-directed) on the biological tissue (e.g. finger).

When the cushion (e.g. **120)** is inflated (i.e. at least partially inflated) (e.g. via tube **124)** so that a portion of a flexible barrier (e.g. a FOT barrier portion) (e.g. a portion of inner ring **104)** inwardly applies pressure upon the user's finger: (i) laser **160** illuminates the user's biological tissue (i.e. skin of the finger in this example) with laser light that is scattered and/or reflected by the biological tissue; (ii) laser light scattered and/or reflected by the biological tissue is received by light detector **170.** As will be discussed elsewhere, in embodiments of the invention, *en route* from laser **160** to the biological tissue the laser light traverses the pressure-applying cushion and/or an interior of the cushion and/or an optically-transparent region of a rigid restrictor (discussed below). As will be discussed elsewhere, in embodiments of the invention, *en route* from the illuminated biological tissue to light detector **170** the reflected and/or scattered laser light traverses the pressure-applying cushion and/or an interior of the cushion and/or an optically-transparent region of a rigid restrictor (discussed below).

As shown in Figs. 2A-2B, the system for optically measuring systolic and/or diastolic blood pressure further comprises blood motion computation circuitry **102** for computing, from laser light reflected by the biological tissue (e.g. finger) and received by light detector **170** a blood-motion signal descriptive of arterial blood motion (e.g. a pulsatile signal). Blood motion computation circuitry **102** may be implemented by any combination of software, hardware (e.g. digital and/or analog) and firmware.

As shown in Fig. 2B, collectively, the combination of laser **160,** light detector **170** and blood motion computation circuitry **102** comprise an optical blood motion sensor **180.** Examples of optical blood motion sensors include laser Doppler sensors , DLS sensors and PPG sensors.

The system for optically measuring systolic and/or diastolic blood pressure further comprises blood pressure circuitry **104** (e.g. implemented by any combination of software, hardware (e.g. digital and/or analog) and firmware) for computing a systolic and/or diastolic blood pressure, from the combination of (i) output of the optical blood motion sensor (e.g. a pulsatile signal computed by the blood motion sensor) and (ii) a measurement of pressure applied by the inflatable cushion or cuff upon the biological tissue (e.g. skin of the finger). - for example, from a time-correlation between the optical blood motion sensor and the pressure measurement.

In some embodiments, the pressure applied by the inflatable cushion or cuff upon the biological tissue (e.g. by FOT barrier portion thereof of the cushion) may be measured according to a measurement of an internal pressure inside the inflatable cushion. Towards this end, structures where the blood motion sensor **180** is situated to avoid mechanical interference (i.e. non-contact configuration - for example, sensor **180** and tissue **159** are on opposite sides of a rigid restrictor) may be preferred since for these structures the internal pressure inside the inflatable cushion would more accurately matches the pressure applied by the inflatable cushion or cuff upon the biological tissue.

Although it may be preferred to measure applied pressure by measuring the internal gas or internal liquid pressure (i.e. pressure of gas or liquid disposed within the cushion) within the cushion **120,** this not a requirement. Alternatively, this measurement of pressure applied upon the biological tissue by the inflatable cushion (e.g. by FOT barrier portion thereof) may be obtained directly - e.g. by measuring directly force applied on the surface of the biological tissue using, for example, using a strain gauge and dividing this measured force by an applied area.

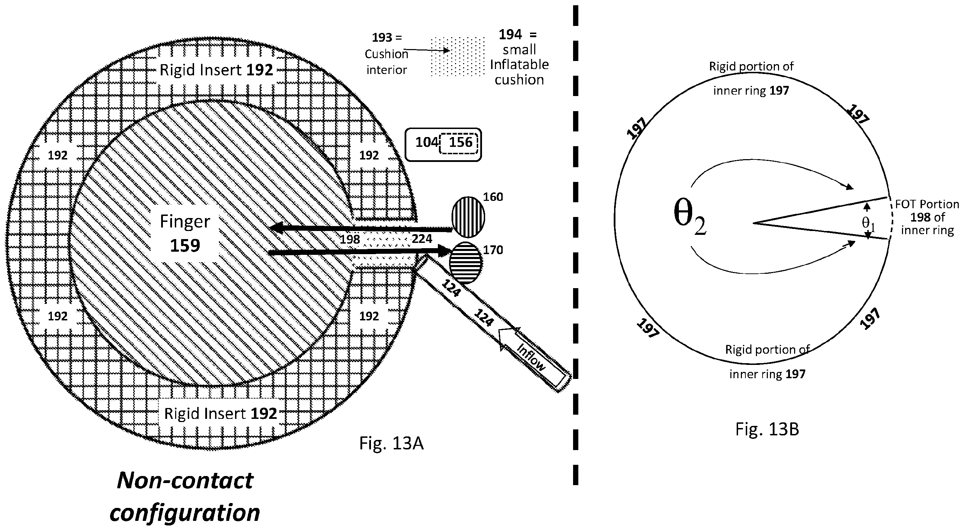

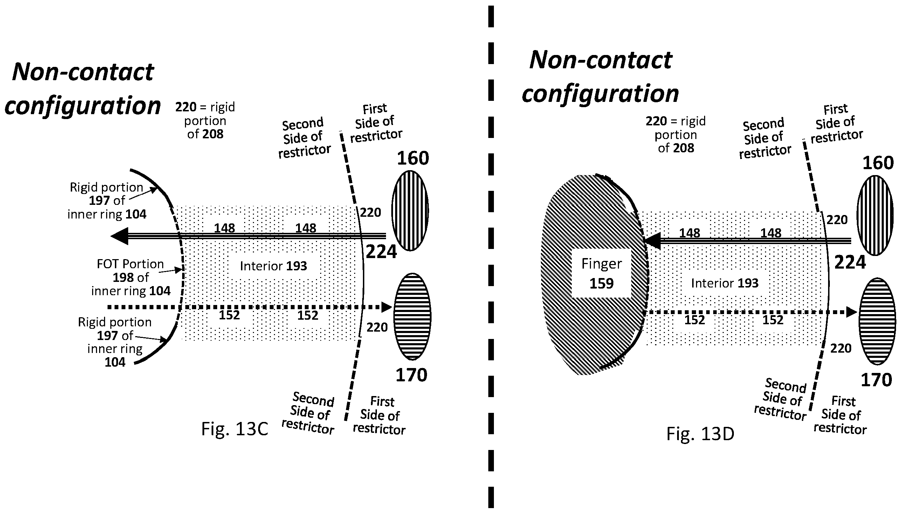

As noted above, the system comprises arterial blood pressure circuitry **104** - in one particular non-limiting embodiment, blood pressure circuitry **104** optionally operates according to the procedure disclosed with reference to Figs. 13-14. For example, blood pressure circuitry **104** may optionally include pulse-wave-form (PWF) scoring engine **156.**

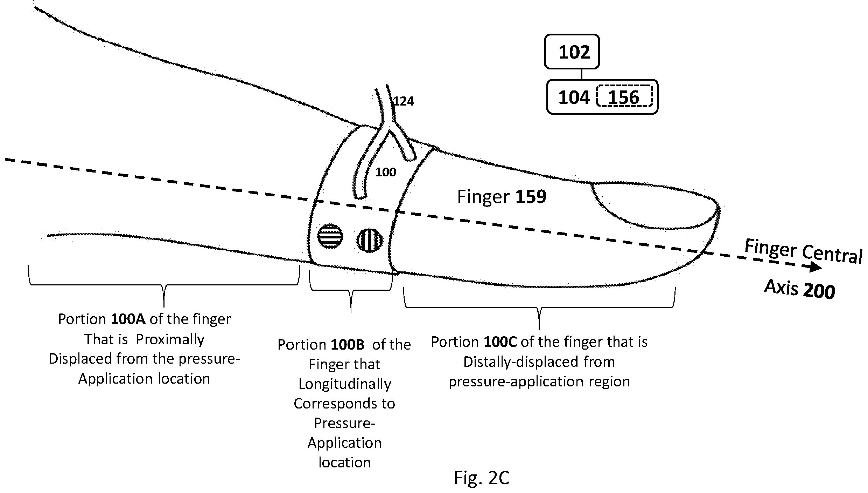

Fig. 2C is in contrast to the example of Fig. 1. As shown in Fig. 2C, the optical measurement of blood motion may be performed on a portion **100B** of the finger that longitudinally (i.e. along a finger central axis **200)** corresponds to a pressure-applying location. In contrast, in the example of Fig. 1, the optical measurement of blood motion is performed on a portion **100B** of the finger is distally displaced from pressure-applying location. Not wishing to be bound by theory, it is believed that this allows for a more accurate measurement of systolic and/or diastolic blood pressure since the biological tissue is optically probed at a location where the physiological conditions inducted by mechanical pressure (i.e. by inflatable cuff and/or cushion) upon the biological surface prevail.

Also shown in Fig. 2A is portion **100A** of the finger is proximally displaced from pressure-applying location.

Fig. 3A is another illustration of ring assembly **100.** Fig. 3B is a cross-section. As shown in Fig. 3B, the ring assembly **100** includes nested inner **104** and outer **108** rings (e.g. having a common centroid **158)** disposed around a central axis, **298** (e.g. centroid **158** is on central axis **298)** the inner ring **104** comprising a section that is flexible and optically-transparent (FOT), the outer ring **108** comprising a rigid section The outer **108** and inner **104** rings defining the following three regions: i. an innermost region **168** within the inside of the inner ring **104** (e.g. through which the user's finger passes - e.g. substantially aligned with axis **298);** ii. an annular-shaped mediating region **188** outside of the inner ring **104** and within the outer ring **108** (e.g. where an interior of a gas-sealed inflatable cushion and/or chamber is disposed); iii. an outermost region **198** exterior to the outer ring **108** (e.g. where at least a portion of blood motion sensor **180** is located - e.g. at least laser **160** and/or detector **170).**

Fig. 4E illustrates blood-motion sensor **180** or portion **180'** thereof (e.g. at least the laser **160** and/or at least the light detector 170) disposed within housing **1990** (e.g. a chip housing at least laser **160** and/or light detector **170).** In the example, housing include an optical transparent portion **1992** (e.g. constructed of optically optically-transparent material and/or provided as a recess or void in housing 1990). For example, housing 1990 serves to provide a gap between the inflated or inflatable cushion and a light-emitting surface of the laser.

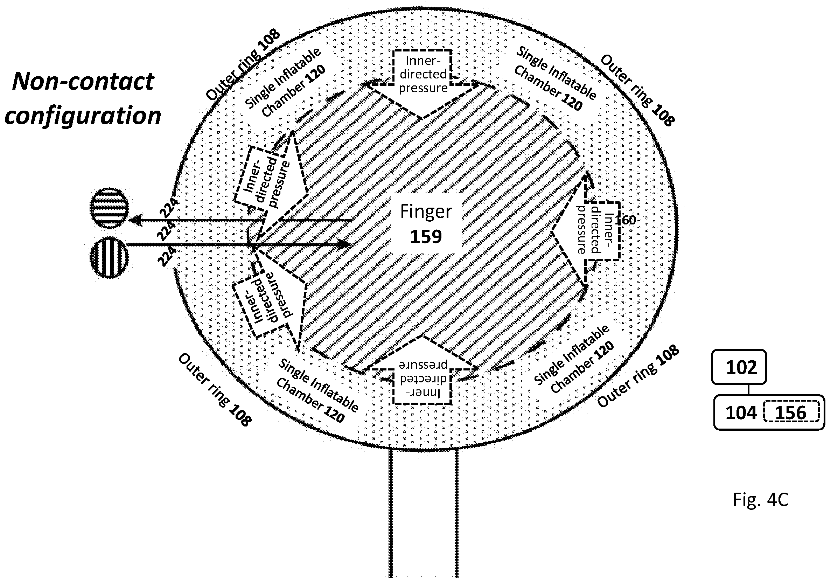

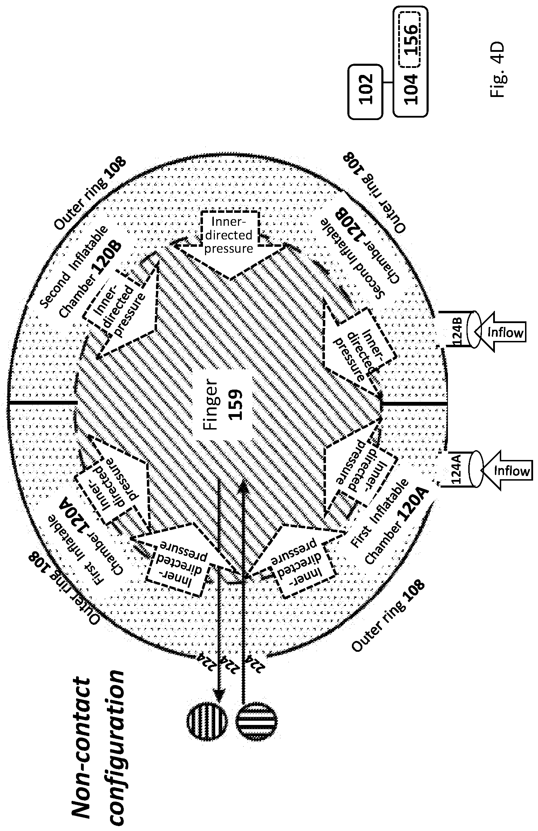

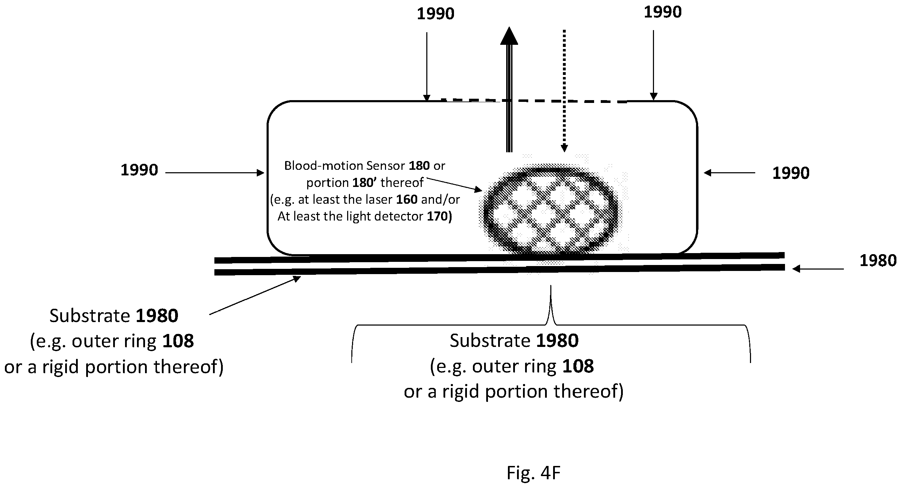

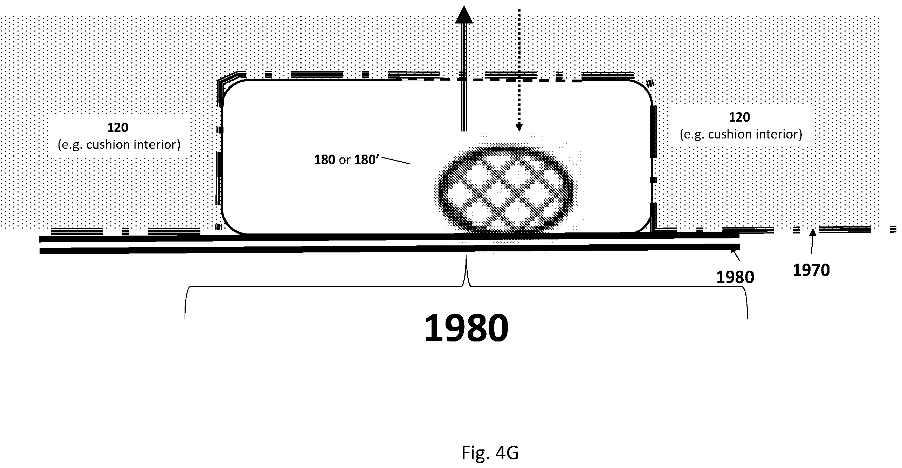

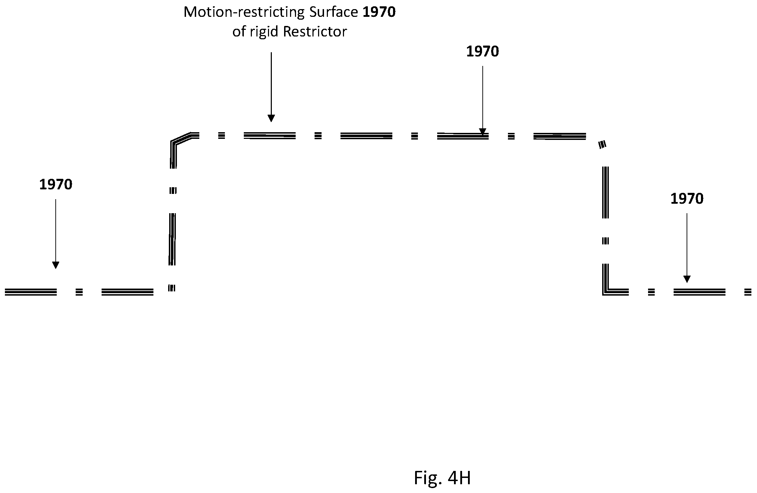

Fig. 4F illustrates the Housing 1990 of Fig. 4 mounted onto a (e.g. rigid substrate) substrate 1980 (e.g. outer ring 108 or a rigid portion thereof). In some embodiments, a portion or entirety of the outer ring and/or of a substrate by itself is the rigid restrictor.

In other embodiments, a portion or entirety of a substrate (e.g. outer ring) together with a portion (e.g. at least 1992) or entirety of a housing (e.g. 1990) collectively comprise the rigid restrictor.

Fig. 4G-4H illustrates motion-restricting Surface 1970 of rigid restrictor defined according to the configuration of Fig 4F. The Motion-restricting Surface 1970 is illustrated using the long dash dot line-pattern
Outer Ring **108** - in some embodiments, at least a portion of the outer ring **108** is rigid. In embodiments of the invention, this outer ring portion function as a restrictor which decreases an amount of time required to inflate an inflatable chamber and/or cushion (not shown explicitly in Fig. 4B) to at least systolic pressure. In some embodiments, a presence of the restrictor (i.e. at least a portion of the outer ring **108** is rigid) is useful or applying a uniform pressure around a circumference of the user's finger. For example, in some embodiments, outer ring **108** comprises one or more of the rigid section(s) which collectively span at least 180 degrees or at least 270 degrees or at least 315 degrees or 330 degrees around the central axis **298.** Although outer ring **108** is drawn as a single section over 360 degrees, it is appreciated that is may be composed of multiple sections and does not need to be 'complete' over the entire 360 degrees.

In various embodiments set forth below, an example will be described where an entirety of the outer ring **108** is rigid. However, this is not a requirement. Although this is not a requirement, providing an outer ring **108** that is entirely rigid may be useful for applying a uniform pressure around the circumference of the finger and/or for minimizing an amount of time required to inflate cushion and/or chamber to inwardly (e.g. from inner ringer **104)** systolic pressure upon the finger disposed in innermost region **168.**

Inner ring **104** -- in some embodiments, at least a portion (e.g. an entirety of) of the inner ring **104** is flexible (e.g. some or all of the inner ring is flexible and optically-transparent (FOT)). For example, interior(s) of one or more gas-sealed inflatable chamber(s) or cushion(s) **120** (not shown in Fig. 3B) may be disposed in mediating region **188,** and the at least a portion of the inner ring may be a portion of a sealing barrier of the inflatable chamber or cushion in mediating region **188.** When the chamber(s) or cushion(s) is inflated, the flexible (e.g. FOT) section of the inner ring applies inwardly-directed pressure upon biological tissue (e.g. finger) disposed in the innermost region **168.** Although inner ring **104** is drawn as a single section over 360 degrees, it is appreciated that is may be composed of multiple sections and does not need to be 'complete' over the entire 360 degrees.

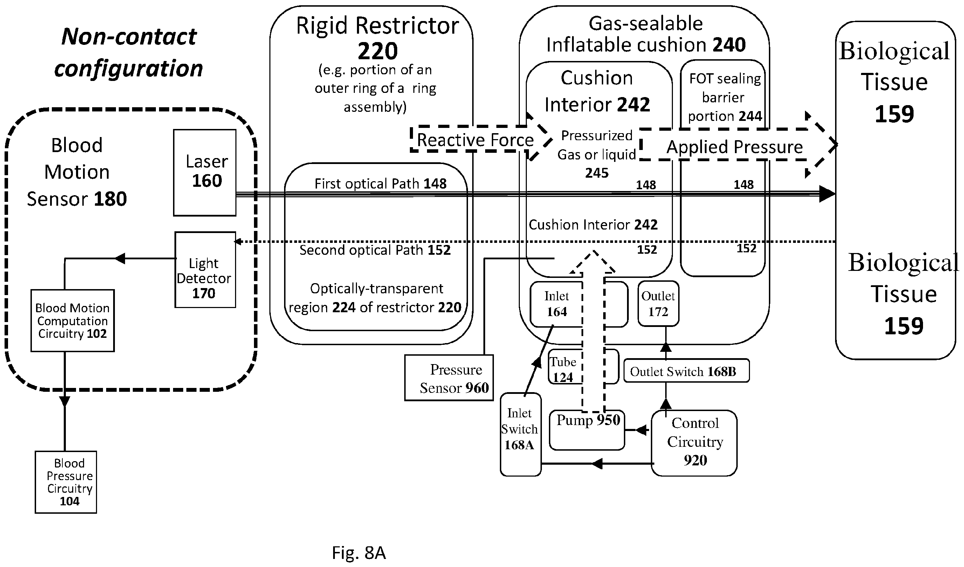

Thus, in different embodiments at least a portion of inner ring **104** is flexible and transparent (FOT). In various embodiments set forth below, an example will be described where an entirety of the inner ring **104** is flexible and/or optically transparent. However, this is not a requirement - see Fig. 8A. Although this is not a requirement, providing an inner ring **104** that is entirely flexible may be useful for applying a uniform pressure around the circumference of the finger. Mediating region **188** -- in embodiments of the invention, respective interiors of one or more a gas-sealable inflatable chambers is(are) disposed in the mediating region between the FOT section of the inner ring and the rigid section of the outer ring (e.g. to collectively span at least 180 degrees or at least 270 degrees or at least 315 degrees or 330 degrees around the central axis **298).** In the example of Fig 4B, a single inflatable chamber **120** occupies an entirety of mediating region **188.** In the example of Fig. 4D, multiple inflatable chambers **120A, 120B** supplied with pressurized gas (or liquid) by tubes **124A, 124B** are shown.

Figs. 4A-4D - As shown in Fig. 4B, when air is forced into chamber **120** via conduit **124,** this forces inward motion of at least a portion of inner ring **204** (e.g. that is a FOT surface of a barrier portion of a cushion and/or chamber **120** within mediating region **188).** This causes the FOT surface to apply inward pressure upon biological tissue **124** (e.g. a finger) disposed within region **168.**

Figs. 4B-4D and 5A-5B, 6, 7A-7C, 8, 9A-9D, 13A and 13C-13D illustrate illumination of finger **159** (i.e. disposed in innermost region **168)** at a time when inflatable cushion/chamber **120** is at least partially inflated (e.g. from tube **124)** to apply inward pressure (e.g. at least systolic pressure) upon the finger.

At this time, laser **160** (e.g. VCSEL) and photodetector **170** of blood motion sensor **180** operate. As will be discussed elsewhere, in different embodiments, blood motion sensor **180** may be a laser Doppler sensor.

In different embodiments and as shown in Fig. 4B and in other figures, (i) no direct contact is required between the laser light source **160** which illuminates the tissue and the skin of the subject (e.g. skin of finger **124)** and/or (ii) no direct contact is required between the light detector **170** which receives light reflected and the skin of the subject. Thus, there may be a 'gap' between the light source and the skin of at least 1 mm or at least 3 mm or at least 5 mm - within this gap is material (i.e. solid, liquid or gas) that is transparent to at least a portion of the visible and/or IR spectrum (i.e. to a wavelength of the light source).

In the example of Figs. 2A-2C, 3A-3B, 4A-4D, 5A-5B, 6, 7A-7C, 9A-9B, 13A, 13C-13D, and 15A, it is possible to locate the light source(s) and/or light detector **170** on a 'outer surface' of outer ring **108** which faces outward and/or away from the subject's skin (and from the innermost region **168)** Alternatively or additionally (NOT SHOWN), it is possible to locate light source(s) and/or light detector in an interior of an inflatable chamber **120** which applies inward pressure on finger **124.** Alternatively or additionally (NOT SHOWN), it is possible to locate light source(s) and/or light detector interior to outer ring **108** but outside of chamber **120** - see, for example, Figs. 9C-9D.

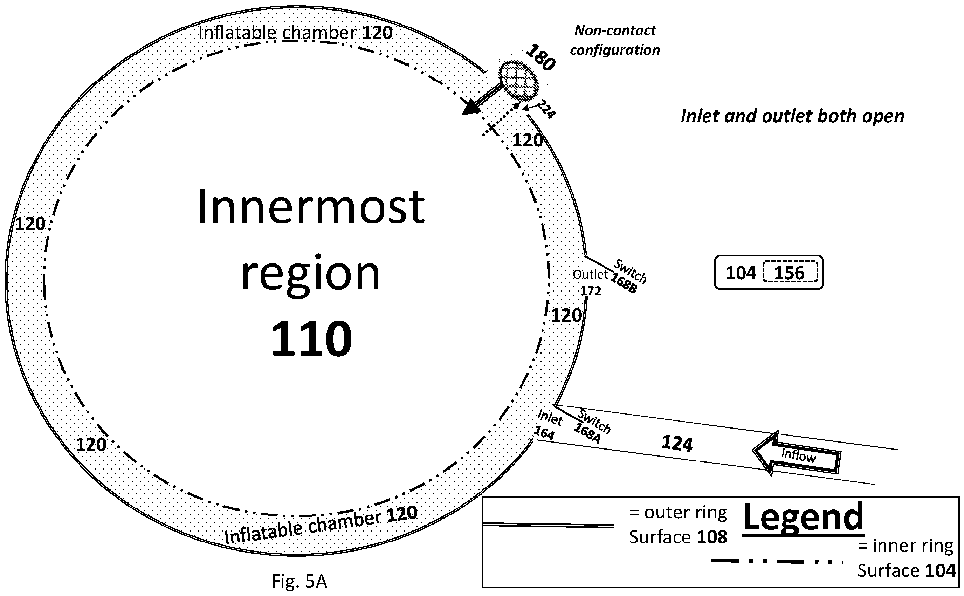

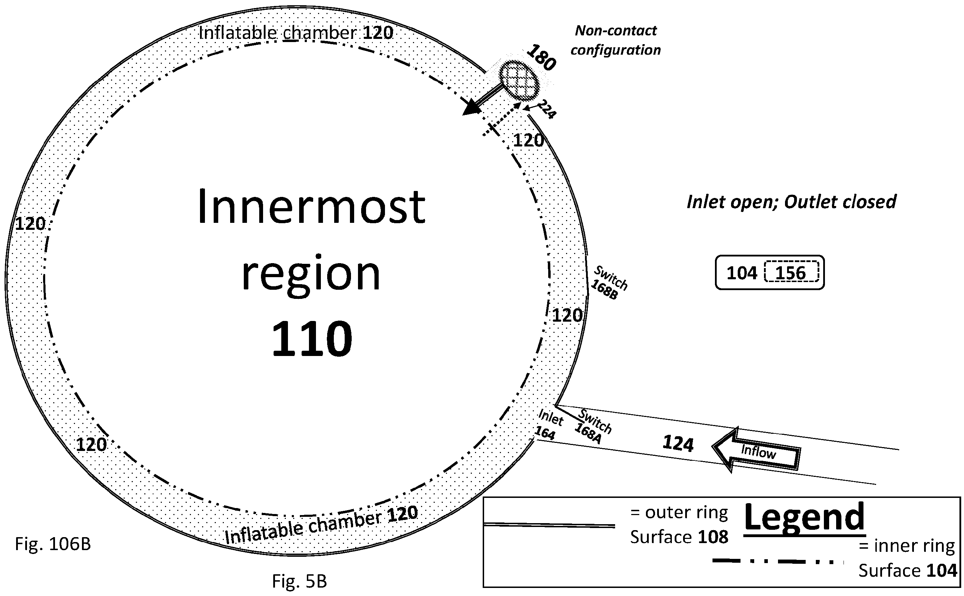

Introduction of pressurized gas (e.g. air) or liquid into chamber **120** (e.g. via pneumatic tube **124** illustrated in Fig. 4A - for example, a pneumatic pump (NOT SHOWN) forces air into chamber **120** via inlet **164** of Fig. 5A) into air-tight inflatable chamber **120** inflates air-tight inflatable chamber **120.** Inflation of the inflatable chamber **120** serves to apply the pressure upon the biological tissue (e.g. finger) in annulus-internal region **110** - for example, an FOT portion of inner ring **104** applies the inward pressure on finger **124.**

In one example, outer ring surface **108** (or at least a portion thereof - e.g. at least 180 degrees around central axis **298)** is rigid and the inner surface **104** (or at least apportion thereof) is flexible - thus, inflation of the chamber **120** causes inward movement (i.e. into and at the expense of annulus-internal region **110)** of inner surface **104** while outer ring surface **108** maintains its dimensions - i.e. introduction of pressurized gas or liquid into chamber **120** does not deform outer ring surface). This combination facilitates application of inwardly-directly pressure that is relatively uniform around the ring. In addition, because the outward ring surface **108** maintains its dimensions, this may be useful for maximizing the inward movement and/or pressure applied upon the biological tissue in annulus-internal region **110** for a given quantity of pressurized gas (e.g. air) or liquid introduced into chamber **120.**

In the examples, an inside of pneumatic tube **124** in fluid communication with an interior of chamber **120** .

In some embodiments, annular shaped ring assembly **100** provides the following features:
(i) at least a portion of the outer **108** surface is optically transparent to at least a portion of the visible and/or IR/ or NIR spectra and (ii) at least a portion of the inner **104** surfaces is optically transparent to at least a portion of the visible and/or IR/or NIR spectra. As such and as illustrated in Fig. 104B, there is: (i) a first optical path **148** between the annulus-internal region **110** and the coherent light source (e.g. VCSEL) of optical sensor **140** via the outer and inner ring surface that is transparent to the portion of the visible and/or IR spectra - i.e. the entirety of the first optical path **148** passes through air (e.g. pressurized air within chamber **120)** or through material that is transparent to the portion of the visible and/or IR spectra; (ii) a second optical path **152** between the annulus-internal region **110** and the light detector source (e.g. VCSEL) of optical sensor **140** via the outer and inner ring surface that is transparent to the portion of the visible and/or IR spectra - i.e. the entirety of the second optical path **152** passes through air (e.g. pressurized air within chamber **120)** or through material that is transparent to the portion of the visible and/or IR spectra,

Illustrated in Fig. 5A-5B is (i) reversibly openable and reversibly closable inlet **164** via which pressurized gas (e.g. air) or liquid is forced into chamber **120** (e.g. via tube **124);** (ii) reversibly openable and reversibly closable outlet **172** via which the gas or liquid leaves chamber **120.** This allows for a 'ramp up' and a 'ramp down' of pressure - e.g. electronic element(s) controls the degree to which inlet **164** is open or closed (e.g. by regulating a position of switch **168A)** and/or electronic element(s) controls the degree to which outlet **172** is open or closed (e.g. by regulating a position of switch **168B).**

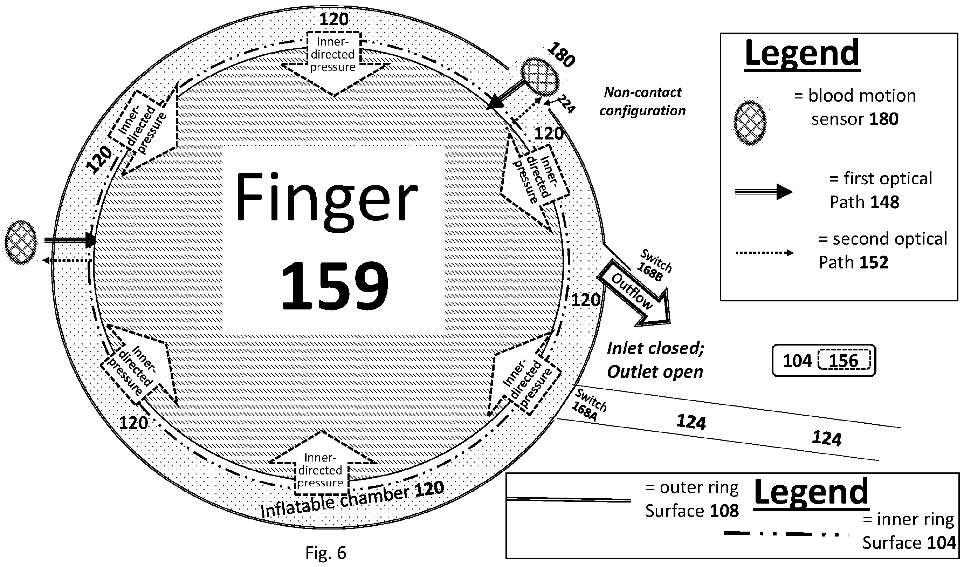

Fig. 6 illustrates an embodiment with multiple blood motion sensors **180** - for example, their signals may be averaged for a more accurate measurement.

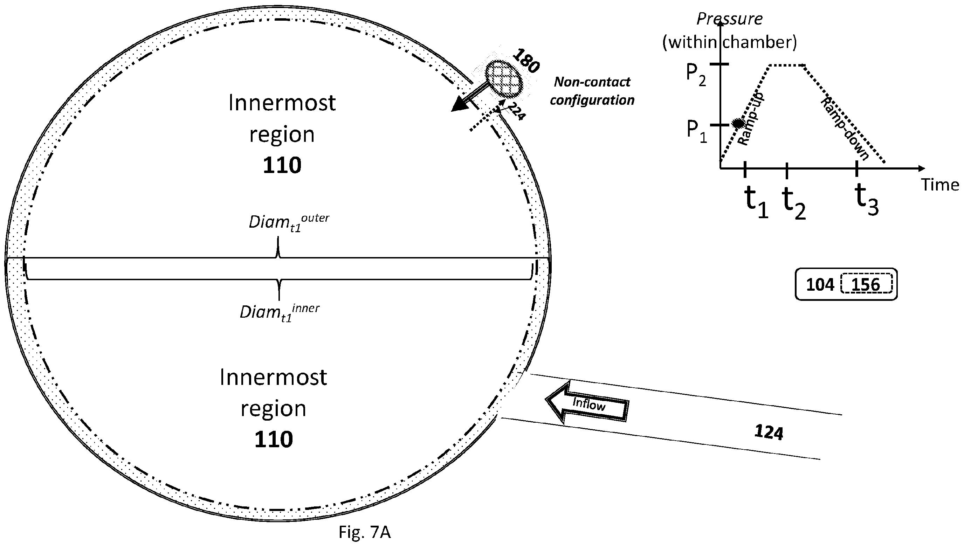

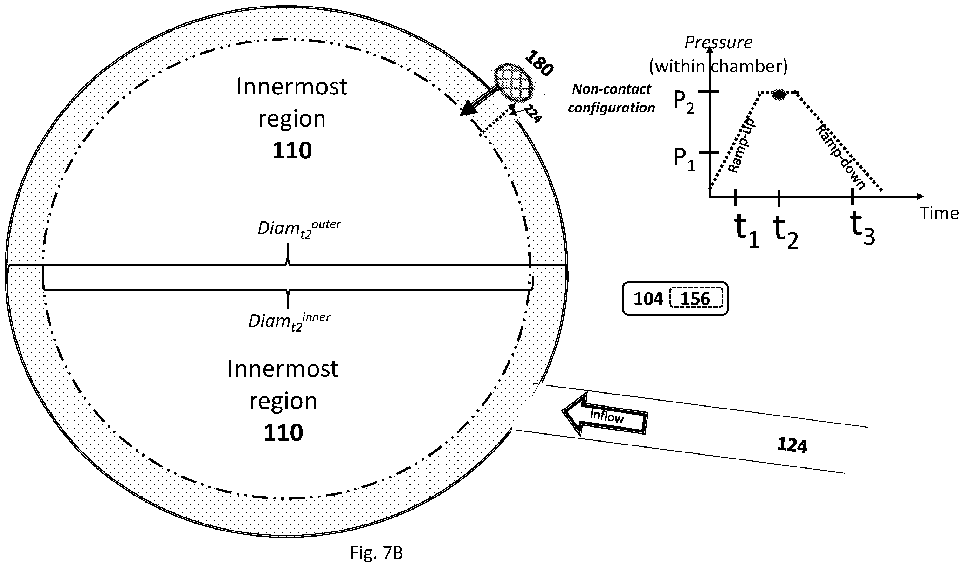

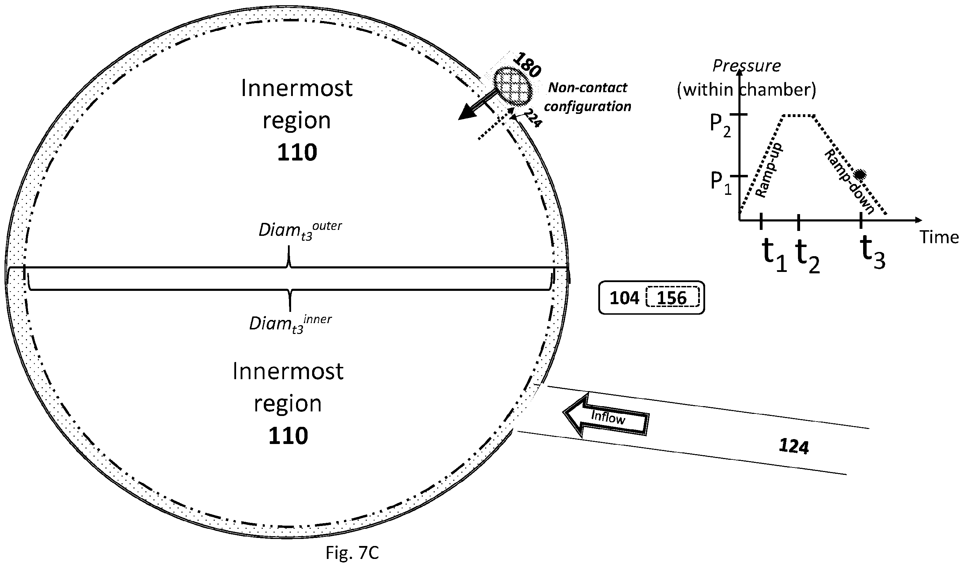

Fig. 7A-7C illustrate pressure ramp-up over time - at time t1 the pressure is relatively low at PI; over time, pressurized gas (e.g. air) or liquid is introduced (e.g. by pump (NOT SHOWN) via **124)** to ramp up the pressure to P2 at time t2 - e.g. switch **168A** keeps inlet open and/or switch **168B** keeps outlet **172** closed. After the pressure increases to P2, outlet **172** is opened to ramp down the pressure within chamber **120.**

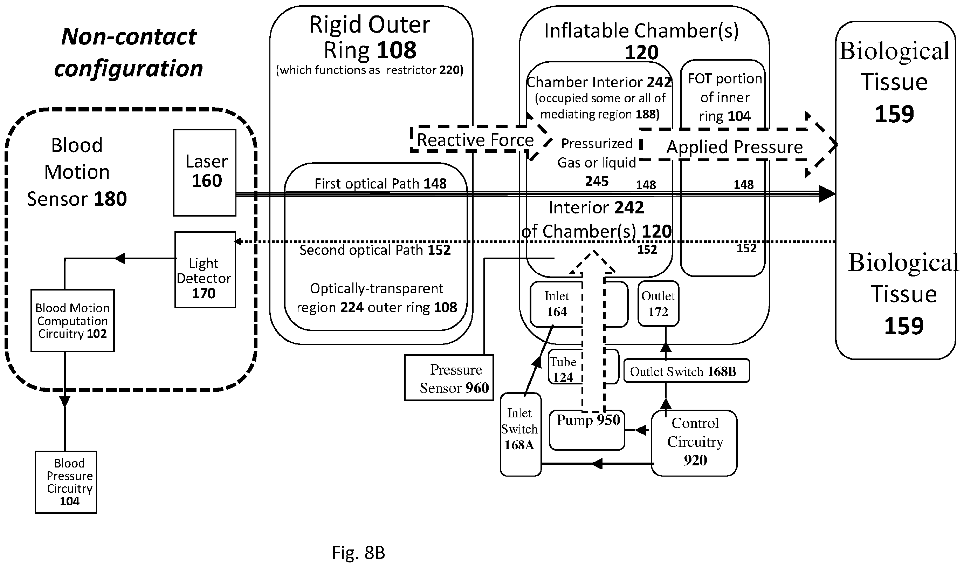

Fig. 8A is a block diagram comprising: (i) an optical blood motion sensor **180** ; (ii) a rigid restrictor **220** (e.g. provided as a portion or an entirety of outer ring **108)** comprising optically-transparent region **224;** (iii) one or more gas-sealable inflatable cushion(s) **240** (e.g. disposed in mediating region **188** between outer **108** and inner **104** rings - e.g. chamber **120);** and (iv) biological tissue Innermost region **168.** Cushion **240** includes a cushion interior **242** where pressurized gas **245** (or liquid) is disposed (e.g. after entering via intel **268A** and tube **124)** As shown in Fig. 8A, this pressurized gas (or liquid) applies pressure upon tissue **159** via FOT barrier portion **244** (e.g. at least a portion of inner ring **104** as in Fig. 8B).

Fig. 8B is implemented according a 'ring assembly form factor' while Fig. 8A relates to the more general case.

In some 'ring assembly' embodiments (e.g. Fig. 8B), (i) locations of the outer ring spanning 360 degrees around central axis **298** are rigid, and at least a portion of that **224** is optically transparent (i.e. void in outer ring **108** or a portion of outer ring **108** constructed of material that is optically transparent); (ii) locations of inner ring **104** spanning around central axis **298** are flexible; (iii) at locations of optical path **148** and **152** inner ring **104** is optically transparent; (iv) locations of mediating region spanning 360 degrees around central axis **298** and spanning the radii between that of the inner ring **104** and that of the outer ring **108** are occupied by a single inflatable chamber **120** or cushion; (v) FOT portions of inner ring **104** serve as a gas-sealing barrier of the inflatable chamber **120** of cushion and are forced inwards upon inflation of the chamber of cushion **120.**

In alternative embodiments different from Fig. 8B, Fig. 8A is implemented according to features of Figs 13A-13B (discussed below) or features of Fig 17 (clip form-factor).

As shown in Fig. 8A laser light from laser **160** follows a first optical path **148** through the optically-transparent region **224** of restrictor **220.** Restrictor **220** may be implemented at 360 degrees of outer ring **20** (which is all rigid around 360 degrees) as in Figs. 3-7, wherein at least portion of the outer ring is optically-transparent region **224.** Although not required, this may be preferably for providing uniform pressure around the circumference of the finger, but it not required.

As shown in Fig. 8A, first optical path **148** also passes through FOT sealing barrier portion **244** of gas-sealable inflatable cushion **240** as well as cushion interior **242** (e.g. in mediating region **188** for ring-assembly embodiments). For example, as is Figs. 3-7, 360 degrees of inner ring 104 may be a flexible (but not necessarily optically-transparent) and serve as a gas-sealing barrier of an annular shaped cushion or chamber **120.**

Figs. 13A-13D relates to alternative embodiments and are discussed below

As shown in Fig. 8A-8B, in some embodiments the air-tight inflatable cushion **240** or chamber **120** has an inlet **164** and an outlet **172.** Also shown is (i) a pneumatic tube **124** in fluid communication with an inside **242** of the air-tight internal chamber via the inlet ; c. a pneumatic pump **950** to inflate the internal air-tight chamber **120** via the pneumatic tube **124** and the inlet **164** so as to cause inward and deforming movement of flexible inner ring surface while the rigid outer ring surface retains its dimensions and shape; d. an electrically controlled intlet switch **168A** for opening and closing the inlet **164;** e. an electrically controlled outlet switch **168B** for opening and closing the outlet **172;** f. pump control circuitry **920** configured to: i. cause the pneumatic pump to inflate the air-tight inflatable chamber, thereby ramping up a magnitude inward-directed pressure applied by the inner ring surface upon annulus-internal-region-disposed-biological tissue; and ii. causing the outlet switch to open the outlet so as to drain air from the air-tight inflatable chamber, thereby ramping down a magnitude of the inward-directed pressure applied by the inner ring surface upon the annulus-internal-region-disposed-biological tissue; g. a pressure sensor **960** for sensing and/or measuring a pressure inside of the air-tight inflatable chamber and/or a pressure sensor for sensing and/or measuring a pressure inside of pneumatic tube **124.**

Blood pressure circuitry **970** computes a blood systolic and/or diastolic blood pressure of the biological tissue by correlating output of the pressure sensor **960** with output (i.e. the results of 'optically sensing pulse' or blood flow) of the optical blood motion sensor **108.**

Reference is now made to Figs. 9A-9B. In embodiments when a rigid restrictor (*e.g.* at least a portion of outer ring **108)** is used, disposing the biological tissue and the laser on the same side of the restrictor (see Figs. 9C-9D where both sensor **108** and tissue **159** are on the 'second ride' of the restrictor - i.e. both are within outer ring **108)** might lead a situation where a presence of laser (or other parts of the optical blood motion sensor **108)** causes 'mechanical interference' - i.e. either uneven application or pressure or a situation where the gas pressure (or pressure of liquid) inside of the cushion does not accurately reflect a magnitude of the pressure applied to the surface.

As such, instead of disposing the biological tissue and the laser on the same side of the restrictor, according to embodiments of the invention, it is preferred to dispose the laser either within inside of the rigid restrictor (e.g. embedded inside) or such (e.g. see Figs. 9A-9B along with Figs. 4B-4D and 6) that laser and the biological tissue are disposed on opposite sides of the rigid restrictor (e.g. the tissue within 'within' ring **108** and the laser **160** and/or detector **170** is outside of ring **108).** As such and as shown Fig. 8A-8B, the rigid restrictor **220** might require the optically transparent region **224** (e.g. either optically-transparent material or a void or window in restrictor **220)** through which laser light passes *en route* to the biological tissue.

As noted above, a presence of the rigid restrictor **22-** serves to reduce the amount of time required to inflate the cushion and/or serves to evenly distribute applied pressure around a circumference of the subject's finger of toe. In some embodiments, (i) blood pressure measurement apparatus comprises a 'ring assembly' cuff assembly ((or ring assembly) having a generally cylindrical shape and disposed around a user's finger or toe; and (ii) the rigid restrictor has a circular shape (or is a rigid portion of an objection that has a circular shape) around the user's finger. In these embodiments, having rigid material at various locations (e.g. all the around) the user's finger is useful for evenly distributing pressure applied to the finger along the finger circumference and/or further reducing the amount of time required to inflate the cushion. In these embodiments, there may be an advantage to a restrictor/ring which is rigid in its entirety - however, even in these embodiments, the present inventors (at present) to do not see any advantage of disadvantage for material of the restrictor to be optically transparent around most or an entire circumference finger - e.g. as long as a region of the rigid restrictor is 'optically transparent.'

In various embodiments (i.e. irrespective of the rigid restrictor), the FOT barrier section (e.g. pressure-applying) and/or an interior of the cushion and/or the optically transparent region of the rigid restrictor serves to provide a gap between (i) the laser of the optical blood motion sensor and (ii) the illuminated biological tissue to prevent contact therebetween. In different embodiments, a thickness of this gap is at least 1 mm or at least 2 mm or at least 3 mm or at least 5 mm.

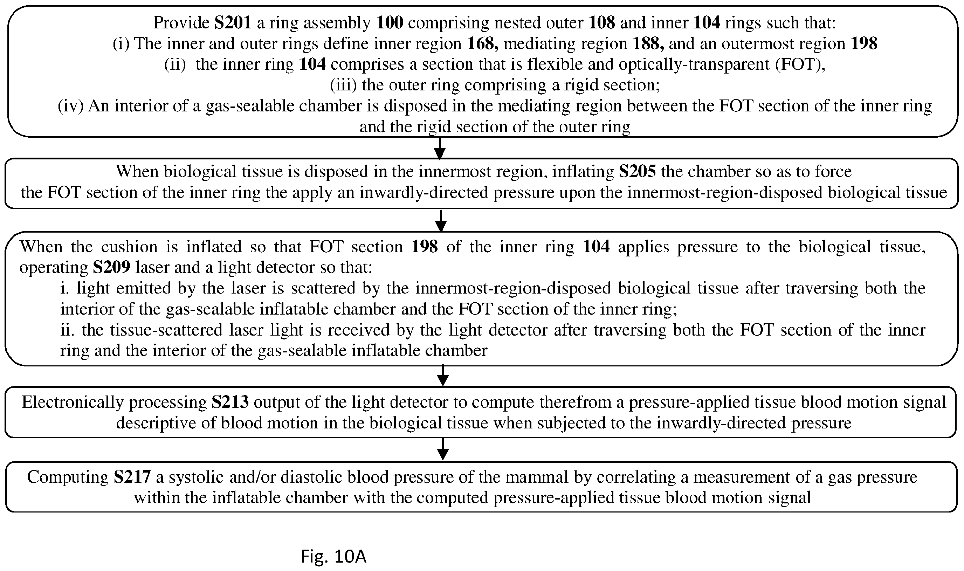

### Discussion of Figs. 10A-10B

Fig. 10A illustrates a method of optically measuring blood pressure of a mammal- e.g. using apparatus of Fig. 8A.

Step **S201** teaches providing **S201** a ring assembly **100** comprising nested outer **108** and inner **104** rings disposed around a central axis, the inner ring comprising a section that is flexible and optically-transparent (FOT), the outer ring comprising a rigid section, the outer and inner rings defining the following three regions: i. an innermost region **168** within the inside of the inner ring **104;** ii. an annular-shaped mediating region **188** outside of the inner ring **104** and within the outer ring **108;** iii. an outermost region **198** exterior to the outer ring **108,** an interior of a gas-sealable inflatable chamber **120** being disposed in the mediating region between the FOT section of the inner ring and the rigid section of the outer ring.

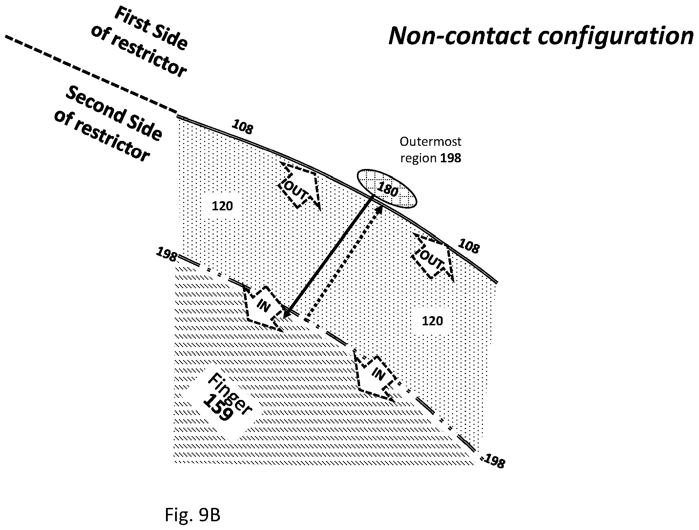

Step **S205** teaches the following: when biological tissue **159** is disposed in the innermost region **168,** inflating the chamber **120** so as to force the FOT section (i.e. at least a portion of **104** - for example, shown in Fig. 9B as **198)** of the inner ring **104** to apply an inwardly-directed pressure upon the innermost-region-disposed biological tissue **159** (e.g. the inward pressure is illustrated in in Fig. 4C)

Step **S209** teaches the following: when the cushion **120** is inflated so that the FOT barrier portion **198** inwardly applies pressure to the biological tissue, operating a laser **160** (e.g. VSCEL) and a light detector **170** so that: i. light emitted by the laser is scattered by the innermost-region-disposed biological tissue after traversing both (i.e. along path **148)** the interior **242** of the gas-sealable inflatable chamber and the FOT section of the inner ring; ii. the tissue-scattered laser light is received by the light detector after traversing (i.e. along path **152)** both the FOT section of the inner ring and the interior of the gas-sealable inflatable chamber;

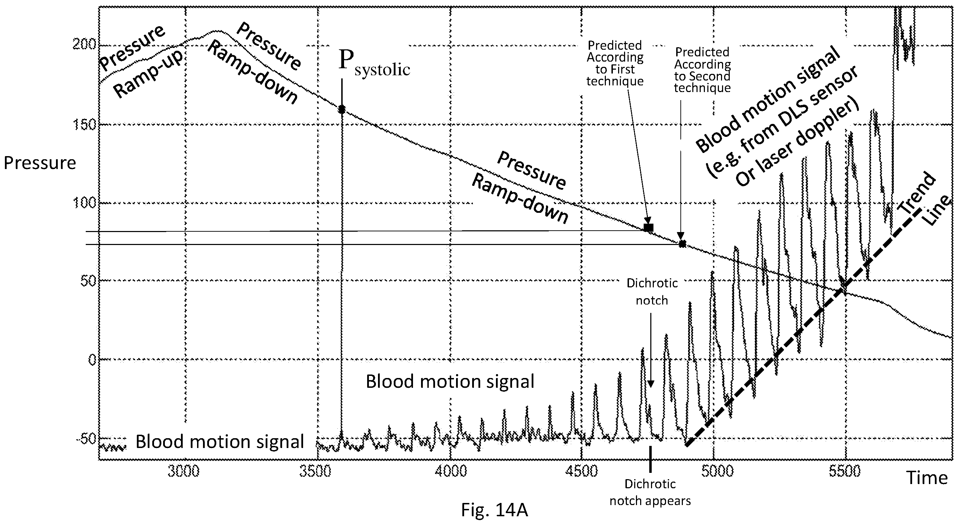

Step **S213** (e.g. performed by blood motion computation circuitry **102)** teaches electronically processing (output of the light detector **170** to compute therefrom a pressure-applied tissue blood motion signal (e.g. see Fig. 14A) descriptive of blood motion in the biological tissue when subjected to the inwardly-directed pressure.

Step **S217** (e.g. performed by blood pressure circuitry **104)** teaches computing a systolic and/or diastolic blood pressure of the mammal by correlating a measurement of a gas pressure or liquid pressure (e.g. output of **960)** within the inflatable chamber with the computed pressure-applied tissue blood motion signal.

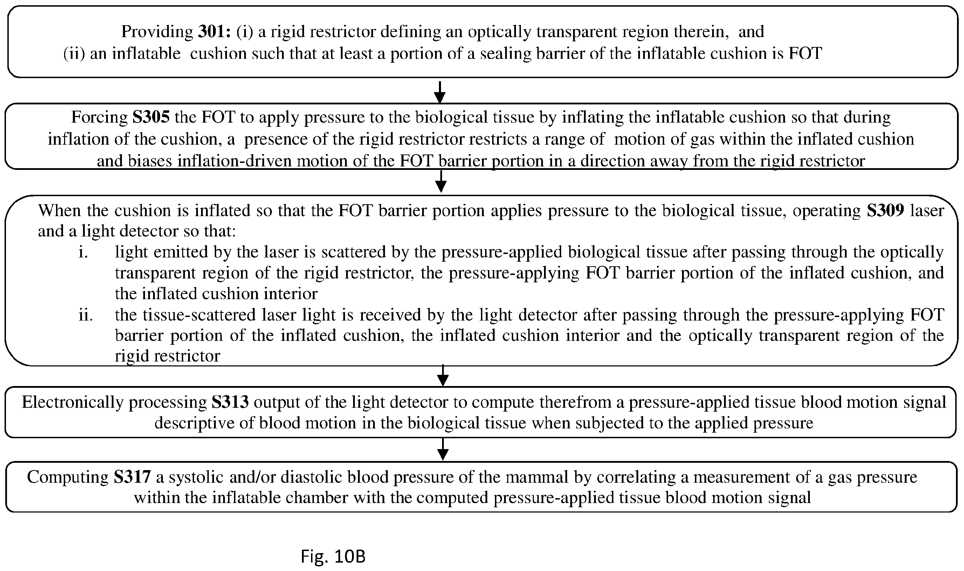

Fig. 10B illustrates a method of optically measuring blood pressure of a mammal having biological tissue - e.g. using apparatus of Fig. 8A or Fig. 8B. For example, a device having a ring assembly or a device with a clip form factor as in Fig. 17.

Step **S301** teaches providing a rigid restrictor **220** (e.g. outer ring **108** or a portion thereof) defining an optically transparent region **224** therein, and an inflatable cushion **240** (e.g. chamber **120),** at least a portion **244** of a sealing barrier of the inflatable cushion (e.g. a portion of inner ring **104)** being flexible and optically transparent (FOT);

Step **S305** teaches forcing the FOT to apply pressure (e.g. inward pressure) to the biological tissue by inflating the inflatable cushion (e.g. via tube **124)** so that during inflation of the cushion, a presence of the rigid restrictor **220** restricts a range of motion of gas (or liquid) within the inflated cushion and biases inflation-driven motion of the FOT barrier portion in a direction away from the rigid restrictor.

As shown in Fig. 9A-9D where ring **108** is rigid and is a restrictor, the restrictor **108** restricts a range of motion of gas (or liquid) within the inflated cushion so that the gas (or liquid) cannot move 'outwardly' into outermost region **198.** As shown in Figs. 9A-9D, pressurized gas **245** (or liquid) within an interior cushion **120** exerts outwards and inwards pressure - the outwards pressure is counteracted by restrictor **108.** Thus, as pressurized gas (or liquid) is introduced into chamber **120** or any other cushion **240,** a presence of restrictor **108** biases inflation-driven motion of the FOT barrier portion in a direction away from the rigid restrictor. In this manner, restrictor **108** functions as a 'contra surface' to the FOT surface (e.g. of inner ring **104)** by applying the reactive force' (see Fig. 8B from **220** to **240)** - this 'contra' or 'reactive force' balances the pressure (i.e. outward pressure in the example of Figs. 9A-9D labelled as 'out') from the pressurized gas (or liquid).

Step **S309** teaches when the cushion is inflated so that the FOT barrier portion applies pressure to the biological tissue, operating a laser **160** (e.g. VCSEL) and a light detector **170** so that: A. light emitted by the laser is scattered by the pressure-applied biological tissue after passing (i.e. along path **148)** through the optically transparent region **224** of the rigid restrictor **220,** the pressure-applying FOT barrier portion **244** (e.g. a portion of inner ring **104)** of the inflated cushion, and the inflated cushion interior **242;** B. the tissue-scattered laser light is received by the light detector **170** after passing (i.e. along path **152)** through the pressure-applying FOT barrier portion **244** of the inflated cushion **240,** the inflated cushion interior and the optically transparent region **224** of the rigid restrictor **220.**

Step **S313** teaches: electronically processing (e.g. by **102)** output of the light detector to compute therefrom a pressure-applied tissue blood motion signal descriptive of blood motion in the biological tissue when subjected to the applied pressure.

Step **S317** teaches computing (e.g. by **104)** a systolic and/or diastolic blood pressure of the mammal by correlating a measurement of a gas pressure (e.g. output of **960)** (or liquid pressure)within the inflated cushion with the computed pressure-applied tissue blood motion signal.

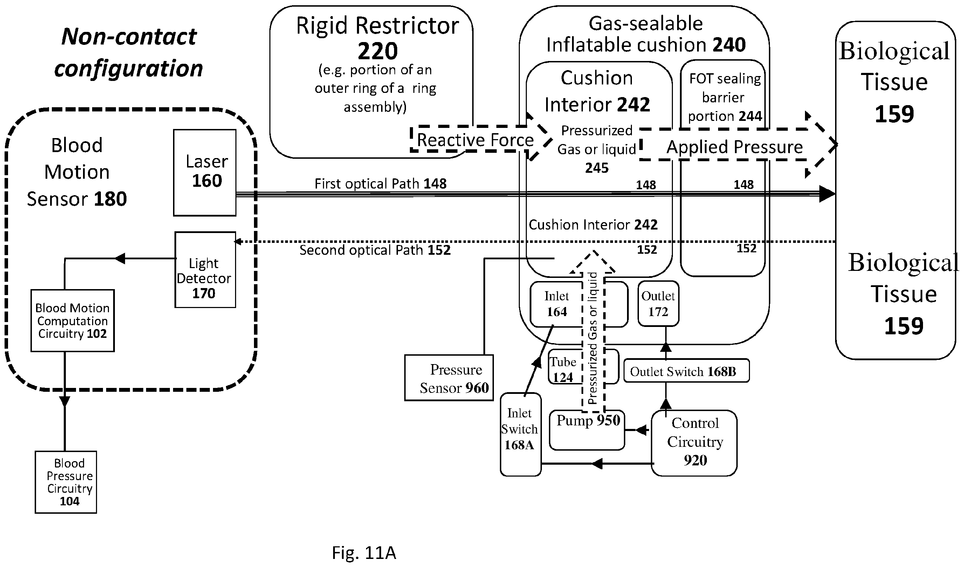

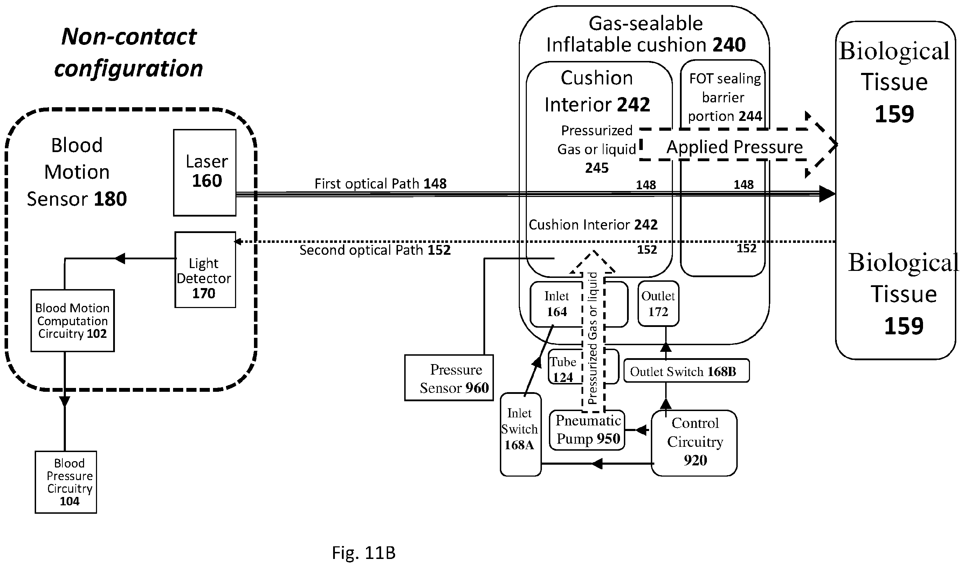

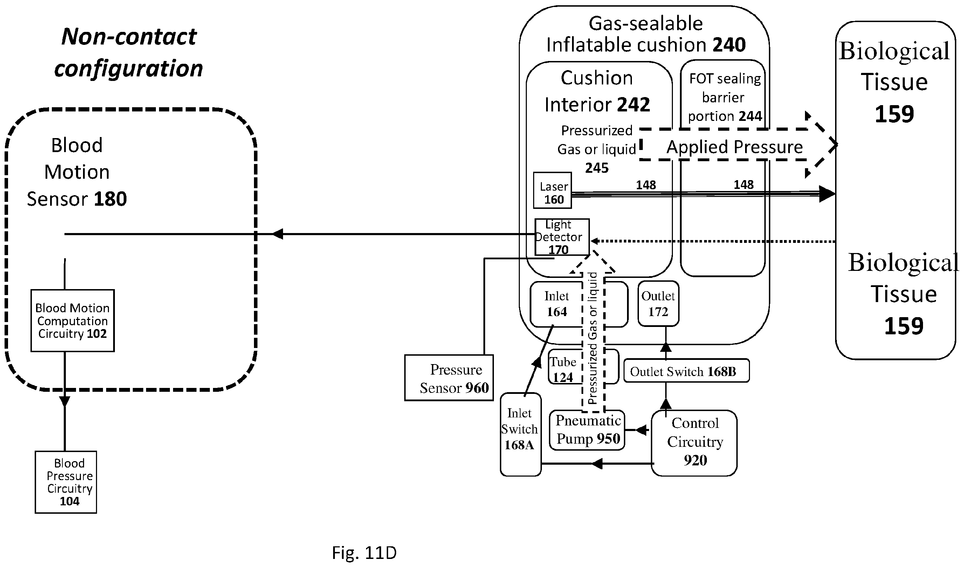

Figs. 11A-11B illustrate alternative embodiments.

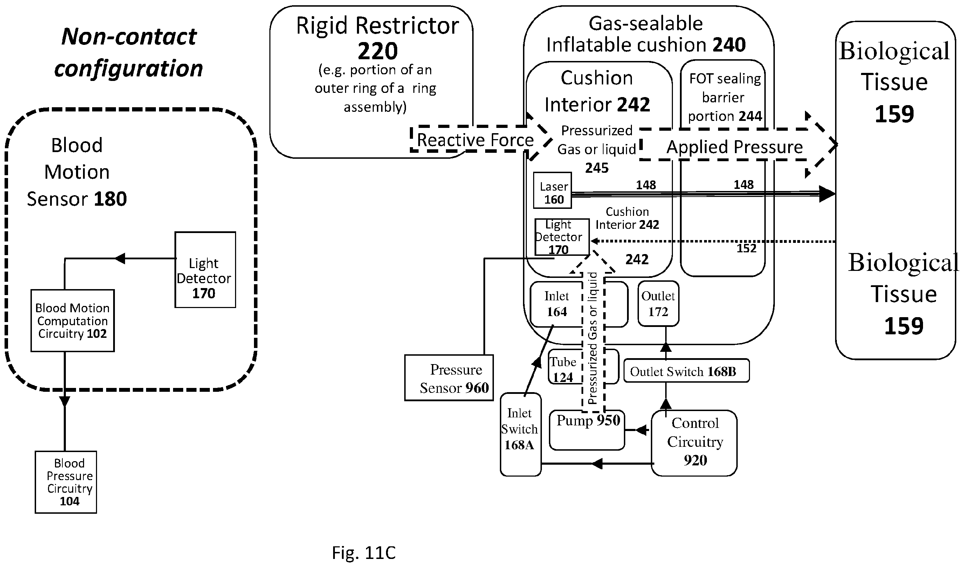

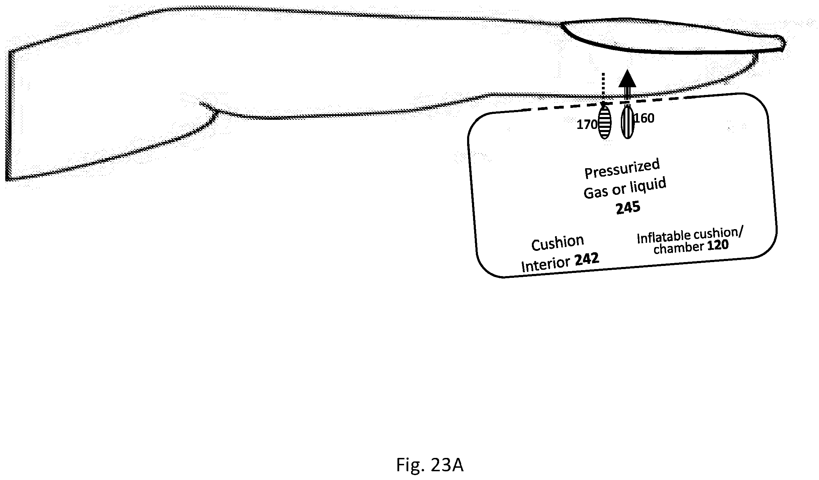

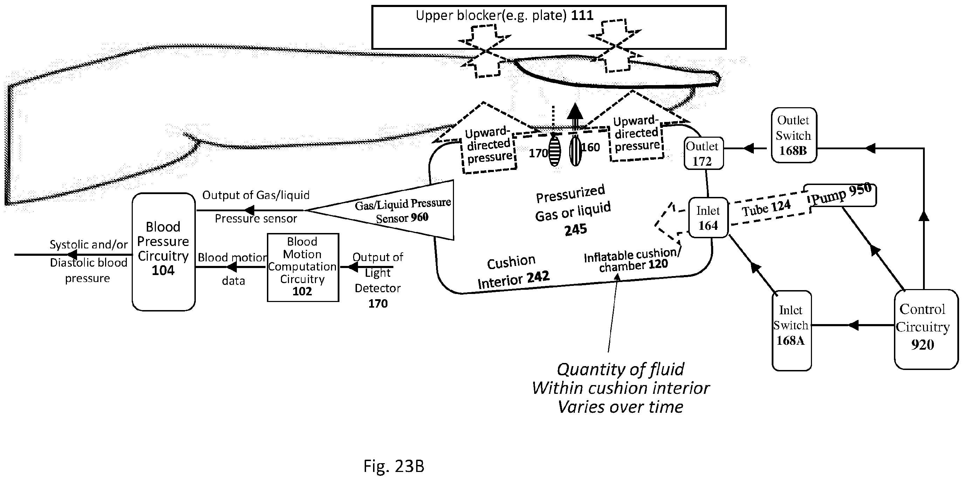

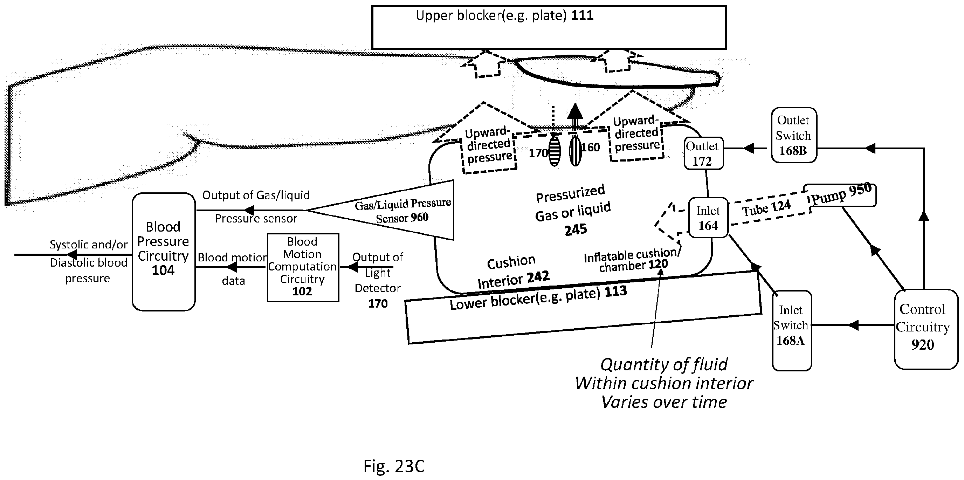

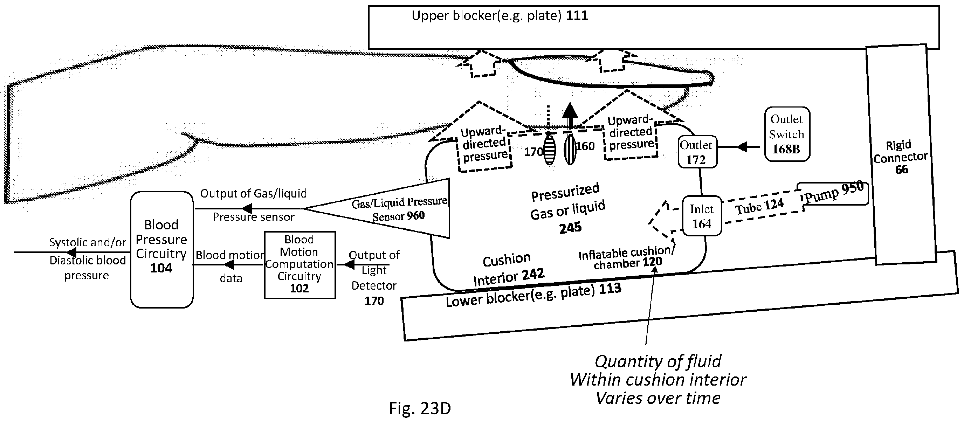

In the examples of Figs. 11C-11D, laser **160** and/or detector **170** are disposed within cushion interior **242.** Figs. 23A-23D illustrate additional examples of embodiments where laser **160** and/or detector **170** are disposed within cushion interior **242.**

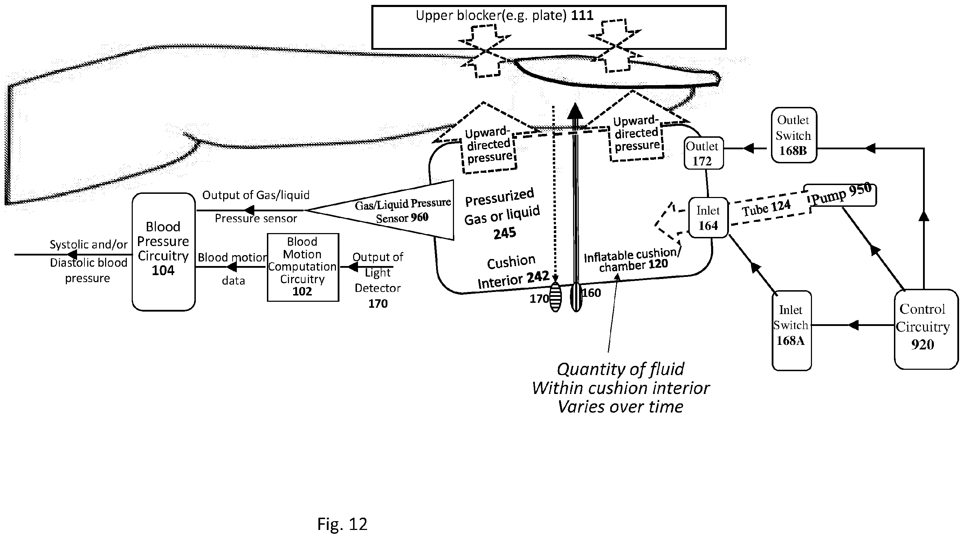

Fig. 12 relates to a situation where both laser **160** and detector **170** are outside of cushion interior **242** (e.g. as illustrated in FIGS. 11A-11B). As pump **950** introduces fluid into an interior of chamber **120,** a distance between laser **160** and the biological tissue increases - for example, the blood motion signal may be acquired for different distances. In some embodiments, this might reduce the accuracy of the blood motion signal and/or the derived systolic and/or diastolic blood pressure measurements.

Alternatively or additionally, this distance may decrease as fluids exits via outlet **172.**

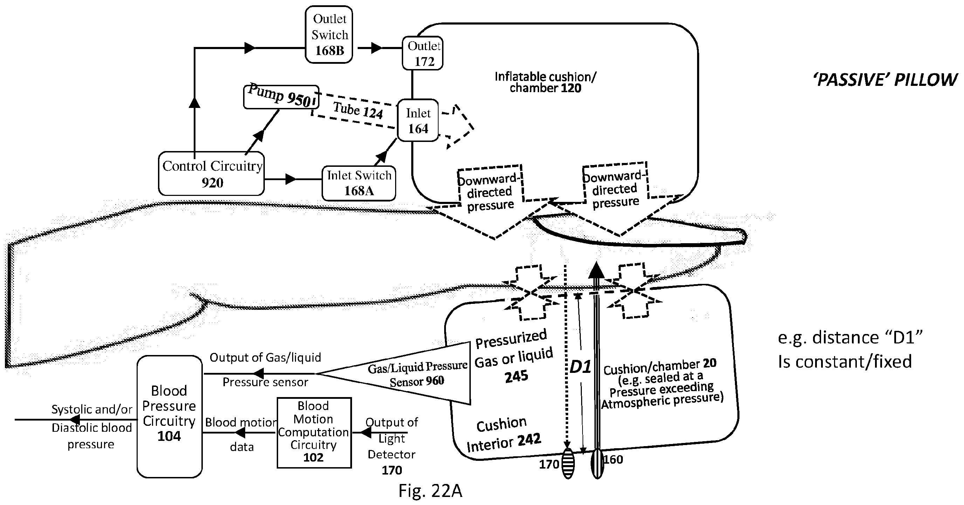

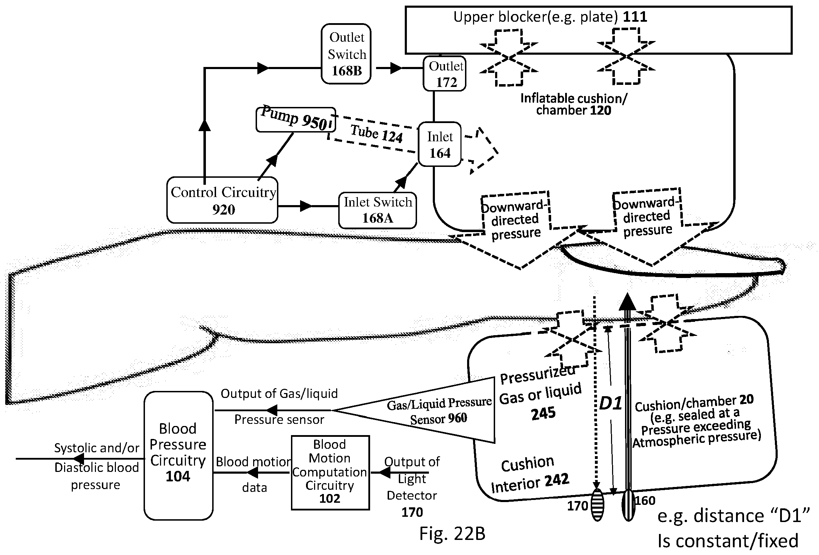

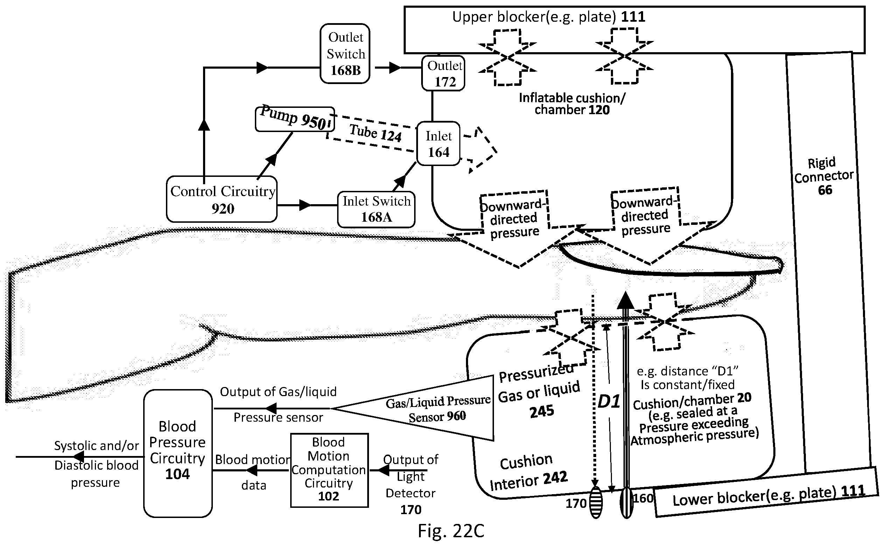

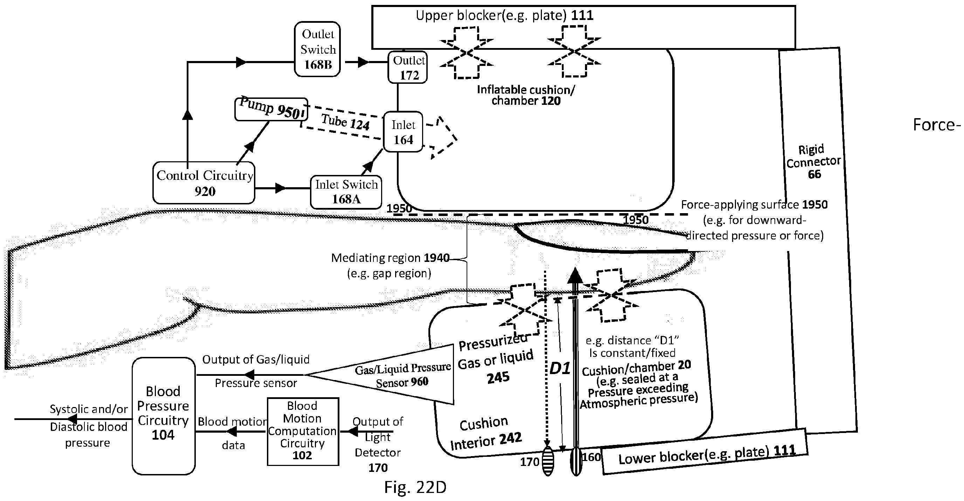

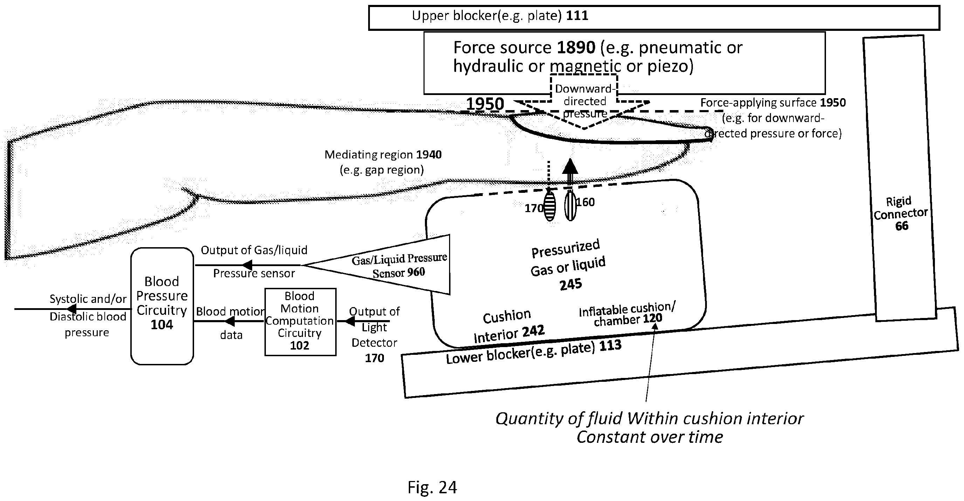

In the examples of Fig. 22A-22D, the no fluid exits or enters cushion interior **242** for different pressures where the blood motion signal is acquired. Thus, in these examples, D1 may remain constant for the different pressures. For example, a separate source of pressure (e.g. pneumatic or hydraulic in FIGS. 22A-22C; for example, not based on pressurized fluid and/or non-pneumatic and non-hydraulic in FIG. 24) may be applied via a force-applying surface **1950** (e.g. for downward- directed pressure or force). This may also obviate the need to provide a hydraulic or pneumatic pump in the product.

In another example (e.g. see FIGS. 23A-23D), pressurized fluid (e.g. gas such as air or liquid) may be introduced or drained for the different pressures where the blood motion signal is acquired. However, by disposing the laser **160** and/or detector **170** are disposed within cushion interior **242** it may be possible to keep the optical paths **148** and/or **152** the length thereof relatively constant, once again avoiding inaccuracies associated with acquiring the blood motion signal for different lengths of the optical paths **148** and/or **152.**

As noted above, in the example of Figs. 3-7, 360 degrees of inner ring **104** may be a flexible (but not necessarily optically-transparent) and serve as a gas-sealing barrier of an annular shaped cushion or chamber **120.** Alternatively, as shown in Figs. 13A-13D , inner ring **104** may have the following feature -- a majority but not all positions around central axis **298** (e.g. subtending θ₂ as in Fig.13B) are occupied by rigid material. Also shown in Fig. 13B is rigid insert **192** which also occupies a majority of mediating region **188** (e.g. subtending θ₂ as in Fig.13B). In the example of Fig. 13A, inward pressure is only applied at locations around central axis **298** spanning a small fraction (e.g. subtending θ₂ as in Fig.1AB) of 360 degrees. Thus may lack some of the advantages of the implementations of Figs. 3-7 (e.g. uniform pressure around a finger or toe circumference) but, nevertheless, for various applications may be sufficient.

### A Discussion of Figs. 13-15

Systolic and/or diastolic blood pressure maybe measured during a 'ramp up' and/or 'ramp down' phase.

Fig. 14A shows two signals plotted together on the same plot - a 'pressure signal' which is the magnitude of pressure applied onto tissue **159** (i.e. including a 'ramp-up' and a 'ramp-down' phase) and blood motion signal. In one example, the systolic pressure is when the blood motion signal becomes 'pulsatile' (e.g. around 3600 in Fig. 14A)

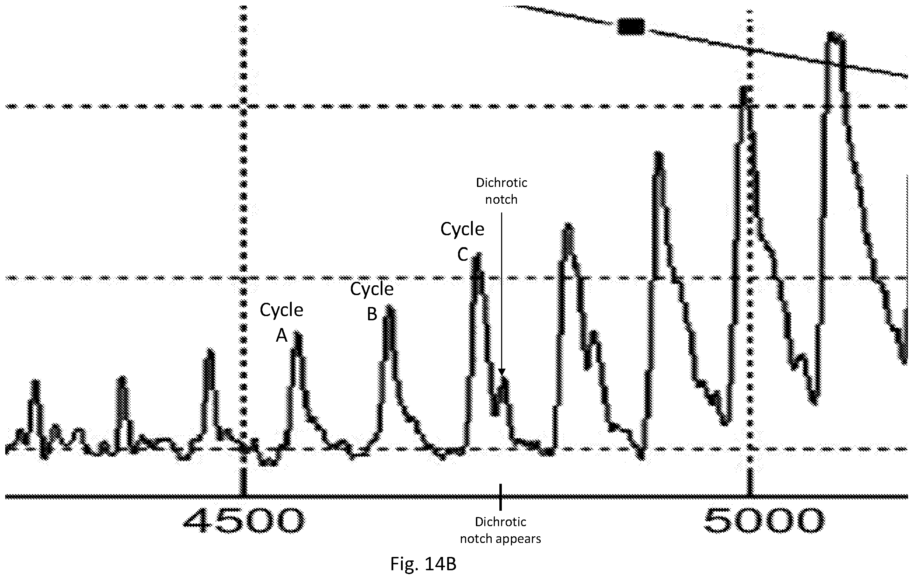

In one example, the diastolic blood pressure may be computed according to the trend line to achieve a result of around 4800 (the 'second technique'). Alternatively or additionally, diastolic blood pressure is achieved by examining a pulsatile wave form feature(s) of blood motion signal - e.g. when dichrotic notch first appears (about 4600) - see Fig. 14B for more details. Thus, for each pulsatile wave, wave form features may be analyzed - thus, as shown in Fig. 2B, blood pressure circuitry **104** may include PWF scoring engine **156.**

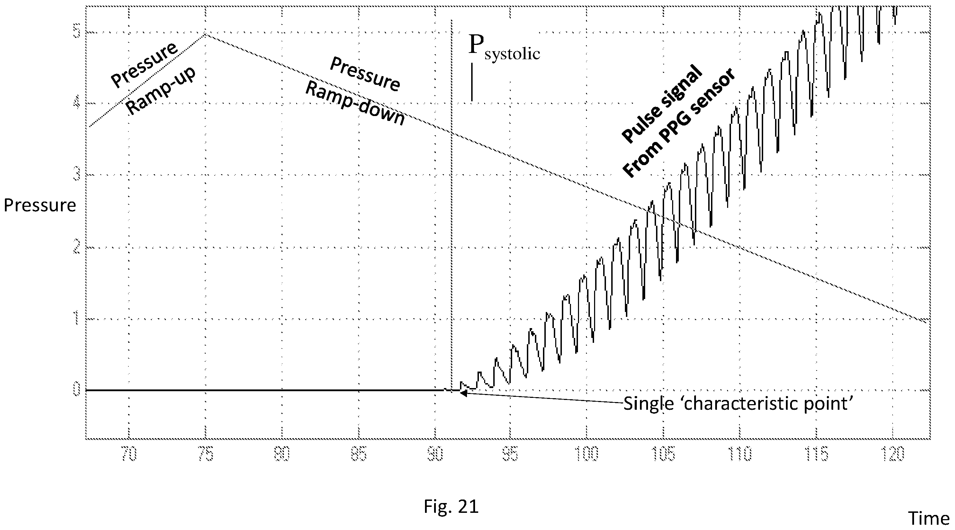

The example of Fig. 14A was obtained using an optical blood pressure detection system where the blood motion sensor **180** is a DLS sensor - it is believed that similar results (i.e. for computing the diastolic blood pressure) may be obtained by employing a laser doppler sensor as blood motion sensor **180.** In contrast (see Fig.21), PPG blood motion signals (i.e. pulsatile signals) lack features for computing diastolic pulse.

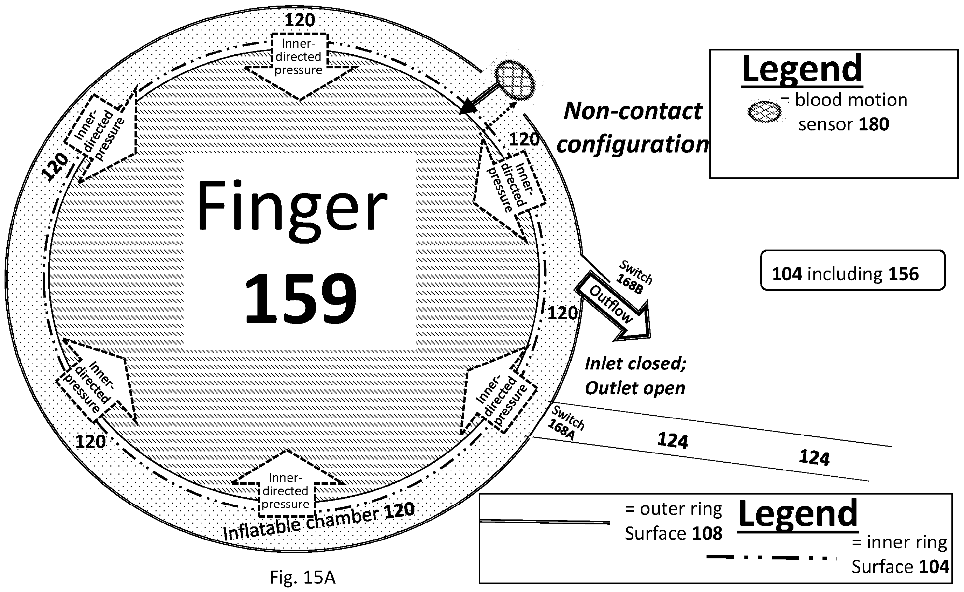

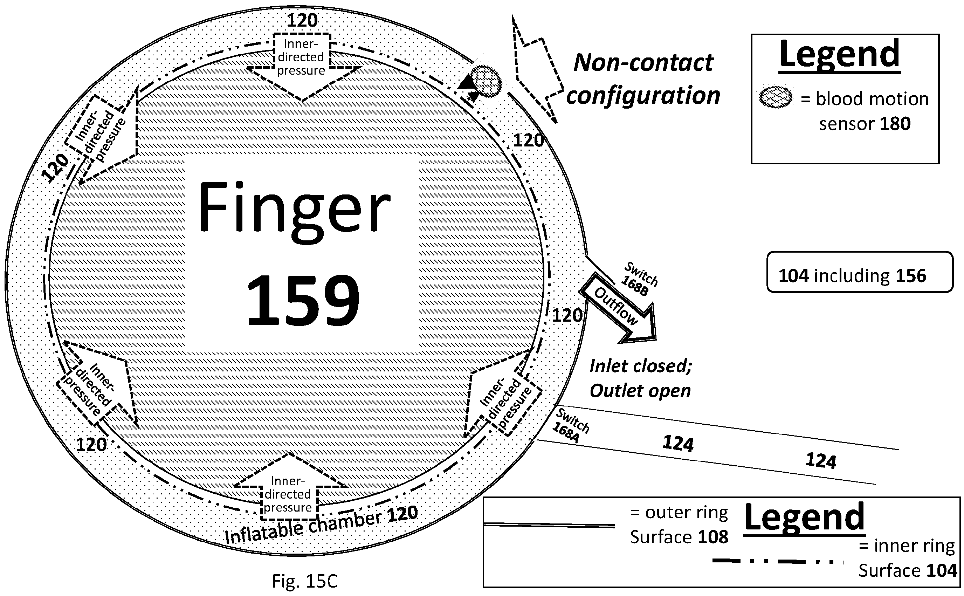

The analysis techniques of Figs. 13-14 may be employed in systems having various mechanical and/or optical properties. Thus, Figs. 15 A and 15C show the non-contact configuration' e.g. to avoid mechanical interference from blood motion sensor **180.** Fig. 15B shows a 'contact configuration' where such problematic mechanical interference might lead to less accurate measurements - e.g. when chamber **120** is inflated inward pressure from the inflated chamber **120** may press sensor **180** against the surface tissue **159,** leading the 'mechanical interference.'

In Fig. 15C sensors **180** may be within chamber **120** or outside of chamber **120** but within ring **108.**

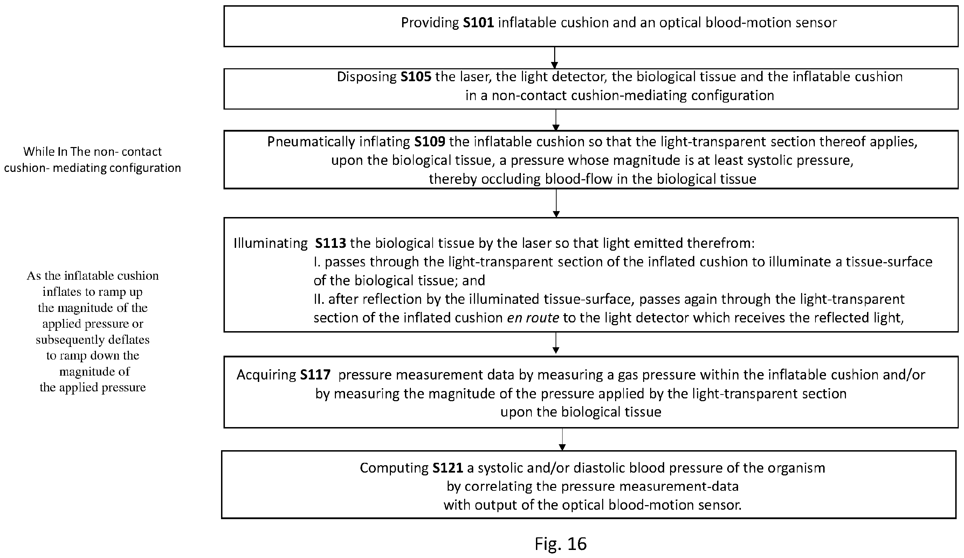

Fig. 16 including steps **S101, S105, S109, S113, S117** and **S121** shows another example.

### A Discussion of Figs. 17A-17D

Although some embodiments relate to a 'ring assembly' form factor, is not a limitation.

Figs. 17A-17D relate to a 'clip form factor' device. Fig. 17C illustrates 'inward' and 'outward' directions. Fig. 17D is the same exact configuration as Fig. 17A but it shows the 'inward' pressure' applied (e.g. on biological tissue) by the FOT portion of the first cushion.

The clip-form-factor device for optically measuring a systolic and/or diastolic blood pressure of a mammal, the system comprising: a. first **320A** and second **320B** rigid plates that are connected to each other to form a V construct so that each plate is respective leg of the V construct, (e.g. the V construct being inwardly biased towards closing the V - for example, the clip-form-factor device is spring loaded), each rigid plate having inward and outward facing surfaces; b. first **240** and second **290** cushions against disposed respectively against inward-facing surfaces **340A, 340B** of the first **320A** and second **320B** legs (e.g. and attached thereto), at least the first cushion **240** being an inflatable cushion, at least a portion **244** of a sealing barrier of the first cushion **240** being flexible and optically transparent (FOT), the first cushion **240** being mechanically coupled to the first rigid plate **320A** (i.e. which functions as a restrictor **220)** so that during inflation of the first cushion **220,** a presence of the first plate restricts a range of motion of gas or liquid within the inflated first cushion and biases inflation-driven motion of the FOT barrier portion in an inward direction away; c. an optical blood-motion sensor comprising a laser and a light detector both of which are attached to the first rigid plate and oriented so that when the first cushion is inflated so that the FOT barrier portion applies inward pressure: A. light emitted by the laser is scattered by the pressure-applied biological tissue the pressure-applying FOT barrier portion of the inflated cushion, and the inflated cushion interior; and B. the tissue-scattered laser light is received by the light the inflated cushion interior and the optically transparent region of the rigid restrictor; iii. output of the light detector is electronically processed to compute therefrom a pressure-applied tissue blood motion signal descriptive of blood motion in the biological tissue when subjected to the applied pressure; and c. blood pressure circuitry configured to compute a systolic and/or diastolic blood pressure of the mammal by correlating a measurement of a pressure within the inflated cushion with the pressure-applied tissue blood motion signal computed by the optical blood-movement sensor.

In the example of Fig. 17A both of the laser and the light detector of the optical blood-motion sensor are disposed between the inward-facing surface of the first plate and the first cushion which is inflatable.

In the example of Fig. 17B, the first plate comprises an optically transparent region, and wherein both of the laser and the light detector of the optical blood-motion sensor are disposed on the outward-facing surface of the first plate so that when the first cushion is inflated so that the FOT barrier portion applies pressure to the biological tissue: A. light emitted by the laser is scattered by the pressure-applied biological tissue after passing through the optically transparent region of the first plate, the pressure-applying FOT barrier portion of the inflated cushion, and the inflated cushion interior; and B. the tissue-scattered laser light is received by the light detector after passing through the pressure-applying FOT barrier portion of the inflated cushion, the inflated cushion interior and the optically transparent region of the rigid restrictor.

Some embodiments relate to a device (e.g. clip-form factor) for optically measuring a systolic and/or diastolic blood pressure of a mammal, the system comprising: a. first and second rigid plates that are mechanically coupled to each other (e.g. connected to each other and/or to define an intermediate region therebetween (e.g. gap between parallel plates or V-interior), each rigid plate having inward and outward facing surfaces; b. first and second cushions against disposed respectively against inward-facing surfaces of the first and second legs, at least the first cushion being an inflatable cushion, at least a portion of a sealing barrier of the first cushion being flexible and optically transparent (FOT), the first cushion being mechanically coupled to the first rigid plate so that during inflation of the first cushion, a presence of the first plate restricts a range of motion of gas or liquid within the inflated first cushion and biases inflation-driven motion of the FOT barrier portion in an inward direction away; c. an optical blood-motion sensor comprising a laser and a light detector both of which are attached to the first rigid plate and oriented so that when the first cushion is inflated so that the FOT barrier portion applies inward pressure: A. light emitted by the laser is scattered by the pressure-applied biological tissue after passing through the pressure-applying FOT barrier portion of the inflated cushion; and B. the tissue-scattered laser light is received by the light detector after passing through the pressure-applying FOT barrier portion of the inflated cushion; iii. output of the light detector is electronically processed to compute therefrom a pressure-applied tissue blood motion signal descriptive of blood motion in the biological tissue when subjected to the applied pressure; and c. blood pressure circuitry configured to compute a systolic and/or diastolic blood pressure of the mammal by correlating a measurement of a pressure within the inflated cushion with the pressure-applied tissue blood motion signal computed by the optical blood-movement sensor.

In some embodiments, the laser and a light detector respectively having light-emitting and light-detecting surfaces, at least one of which is disposed in the cushion interior.
In some embodiments, the laser has a light-emitting surface such that light emitted by the laser is scattered by the pressure-applied biological tissue after passing through the pressure-applying FOT barrier portion of the inflated cushion and the inflated cushion interior.

### A Discussion of FIGS. 23A-23D and 24

Fig. 23A-23D and 24 relate to a device for optically measuring a systolic and/or diastolic blood pressure of a mammal, the system comprising: a. an inflatable cushion **120** having a sealing barrier, at least a portion of which the sealing barrier both flexible and optically transparent (FOT); b. an optical blood-motion sensor **180** comprising a laser and a light detector respectively having light-emitting and light-detecting surfaces, each of the light-detecting and light-emitting surfaces being: i. disposed within an interior **242** of the inflatable cushion **120** (e.g. to one side thereof) and ii. oriented so that when the cushion is inflated so that the FOT barrier portion of the inflated cushion applies pressure upon the biological tissue and/or vice versa: A. light emitted by the laser is scattered by the biological tissue after passing through a section of FOT barrier portion where the FOT barrier portion applies pressure upon the biological tissue and/or vice versa; and B. the tissue-scattered laser light is received by the light detector at the light-detecting surface thereof after passing through the section of FOT barrier portion where the FOT barrier portion applies pressure upon the biological tissue and/or vice versa; iii. output of the light detector is electronically processed to compute therefrom an tissue blood motion signal descriptive of blood motion in the biological tissue when the FOT barrier portion applies pressure upon the biological tissue and/or vice versa; and c. a pressure sensor **960** for performing a measurement of a pressure within the cushion when the cushion is inflated; d. blood pressure circuitry configured to compute a systolic and/or diastolic blood pressure of the mammal by correlating the measurement of the pressure within the inflated cushion with the tissue blood motion signal computed by the optical blood-movement sensor.

In some embodiments, further comprising a (e.g. pneumatic or hydraulic) pump configured to inflate the inflatable cushion to vary an interior pressure of the cushion (see Figs. 23A-23D but not Fig. 24).

In some embodiments, further comprising (see Figs. 23A-23D but not Fig. 24) wherein the tissue blood motion signal is computed for a plurality of interior cushion pressures, each interior cushion pressure associated with a different point in time as the pump forces pressurized fluid into the cushion interior to increase the interior pressure thereof, and wherein the blood pressure circuitry computed the systolic and/or diastolic blood pressure from the tissue blood motion signal for the plurality of interior cushion pressures.

In some embodiments (see Figs. 23A-23D but not Fig. 24). further comprising a reversibly openable and closable outlet such that when the outlet is open that cushion is not sealed to allow pressurized fluid within the cushion interior to exit from the cushion interior, and wherein the tissue blood motion signal is computed for a plurality of interior cushion pressures, each interior cushion pressure associated with a different point in time as the pressurized fluid exits from the interior of the inflatable cushion via the outlet, and wherein the blood pressure circuitry computed the systolic and/or diastolic blood pressure from the tissue blood motion signal for the plurality of interior cushion pressures.

Fig. 24 relates to a device for optically measuring a systolic and/or diastolic blood pressure of a mammal, the system comprising: a. a sealed and pressurized (SAP) cushion **120** having an internal pressure (e.g. of gas such as air) exceeding atmospheric pressure, the sealed cushion **120** having a sealing barrier, at least a portion of which the sealing barrier both flexible and optically transparent (FOT); b. an optical blood-motion sensor **180** comprising a laser and a light detector respectively having light-emitting and light-detecting surfaces, each of the light-detecting and light-emitting surfaces being: i. disposed within an interior **242** of the inflatable cushion **120** (e.g. to one side thereof) and ii. oriented so that when the cushion is inflated so that the FOT barrier portion of the SAP cushion applies pressure upon the biological tissue and/or vice versa: A. light emitted by the laser is scattered by the biological tissue after passing through a section of FOT barrier portion where the FOT barrier portion applies pressure upon the biological tissue and/or vice versa; and B. the tissue-scattered laser light is received by the light detector at the light-detecting surface thereof after passing through the section of FOT barrier portion where the FOT barrier portion applies pressure upon the biological tissue and/or vice versa; iii. output of the light detector is electronically processed to compute therefrom an tissue blood motion signal descriptive of blood motion in the biological tissue when the FOT barrier portion applies pressure upon the biological tissue and/or vice versa; and c. a pressure sensor **960** for performing a measurement of a pressure within the cushion when the cushion is inflated; d. blood pressure circuitry configured to compute a systolic and/or diastolic blood pressure of the mammal by correlating the measurement of the pressure within the inflated cushion with the tissue blood motion signal computed by the optical blood-movement sensor.

In some embodiments (e.g. see Fig. 24), the sealed and pressurized cushion is permanently sealed.

In some embodiments, further comprising force source (e.g. see Fig. 24) having a force-applying surface **1950** that is displaced from the FOT.

In some embodiments (e.g. Fig. 24), configured to measure the blood pressure while a quantity of fluid within the SAP cushion or while a pressure within the cushion remains constant.

In some embodiments, (any embodiment) further comprising a force source having a force-applying surface that is outside of the SAP cushion to device a mediating **1940** (e.g. gap region) region in between the FOT barrier portion and the force-applying surface, the force source configured, when the biological tissue is disposed in the mediating (e.g. gap) region, to urge the biological tissue towards the FOT barrier portion so that the biological tissue applies pressure upon the FOT barrier portion.

In some embodiments (e.g. see Figs. 24), the force source is pneumatic or hydraulic.

In some embodiments (e.g. Figs. 24) the force source **1890** is not based upon pressurized fluid (i.e. not pneumatic or hydraulic) - for example, a piezo force source or a magnetic force source.

In some embodiments, at least a portion of the laser is fixedly mounted to an interior of the inflatable or SAP cushion.

In some embodiments, at least a portion of the laser is fixedly mounted to an interior of the inflatable or SAP cushion so that a beam emitted by the light-emitting surface passes through the FOT barrier at a beam-traverse location.

In some embodiments, at least a portion of the laser is fixedly mounted to an interior of the inflatable or SAP cushion so that (i) as the inflatable or SAP cushion is inflated, a distance between the light-emitting surface and the beam-traverse location of the FOT barrier portion is preserved and/or (ii) inflation of the inflatable or SAP cushion serves to move both the beam-traverse location of the FOT barrier and the light-emitting surface of the laser in the same direction.

In some embodiments, at least a portion of the light detector is fixedly mounted to an interior of the inflatable or SAP cushion so a the light scattered by the biological tissue is received by the light-emitting surface of the light detector after passing through the FOT barrier at a scattered-light-traverse location.

In some embodiments, the at least a portion of the laser is fixedly mounted to an interior of the inflatable or SAP cushion so that (i) as the inflatable or SAP cushion is inflated, a distance between the light-emitting surface and the scattered-light-traverse location of the FOT barrier portion is preserved and/or (ii) inflation of the inflatable or SAP cushion serves to move both the scattered-light-traverse location of the FOT barrier and the light-detcting surface of the light detector in the same direction. (e.g. this is pneumatic or hydraulic including pump 950; in Fig. 24 this force source **1890** may be other than hydraulic and other than pneumatic)

### A Discussion of FIGS. 22A-22E and 24

Fig. 22A-22E and 24 relate to a system for optically measuring a systolic and/or diastolic blood pressure of a mammal, the system comprising: a. a sealed and pressurized (SAP) cushion **120** having an internal pressure exceeding atmospheric pressure, the sealed cushion **120** having a sealing barrier, at least a portion of which the sealing barrier both flexible and optically transparent (FOT); b. an optical blood-motion sensor **180** comprising a laser and a light detector respectively having light-emitting and light-detecting surfaces such that: i. at least one (e.g. both of) of the light-emitting surface and the light-detecting surface is mechanically coupled to the sealing barrier of the cushion (e.g. coupled to the FOT portion thereof); and/or ii. at least one of **(e.g. both of)** light-emitting surface and the light-detecting surface is disposed in an interior of the SAP cushion; and wherein the light-emitting surface and the light-detecting surface are disposed so that (i) when the cushion is sealed so that a quantity of fluid in the cushion interior remains constant; and (ii) when FOT barrier portion of the SAP cushion applies pressure upon the biological tissue and/or vice versa: A. light emitted by the laser is scattered by the biological tissue after passing through a section of FOT barrier portion of the sealed cushion where the FOT barrier portion of the sealed cushion applies pressure upon the biological tissue and/or vice versa; and B. the tissue-scattered laser light is received by the light detector at the light-detecting surface thereof after passing through the section of FOT barrier portion of the sealed cushion where the FOT barrier portion applies pressure upon the biological tissue and/or vice versa; iii. output of the light detector is electronically processed to compute therefrom an tissue blood motion signal descriptive of blood motion in the biological tissue when the FOT barrier portion of the sealed cushion applies pressure upon the biological tissue and/or vice versa; and c. a pressure sensor **960** for performing a measurement of a pressure within the cushion when the cushion is inflated; d. blood pressure circuitry configured to compute a systolic and/or diastolic blood pressure of the mammal by correlating the measurement of the pressure within the inflated cushion with the tissue blood motion signal computed by the optical blood-movement sensor.

In some embodiments (e.g. see FIG. 24) further comprising a force source having a force-applying surface that is outside of the SAP cushion to device a gap region in between the FOT barrier portion and the force-applying surface, the force source configured, when the biological tissue is disposed in the gap region, to urge the biological tissue towards the FOT barrier portion so that the biological tissue applies pressure upon the FOT barrier portion.

In some embodiments the force source is pneumatic or hydraulic.

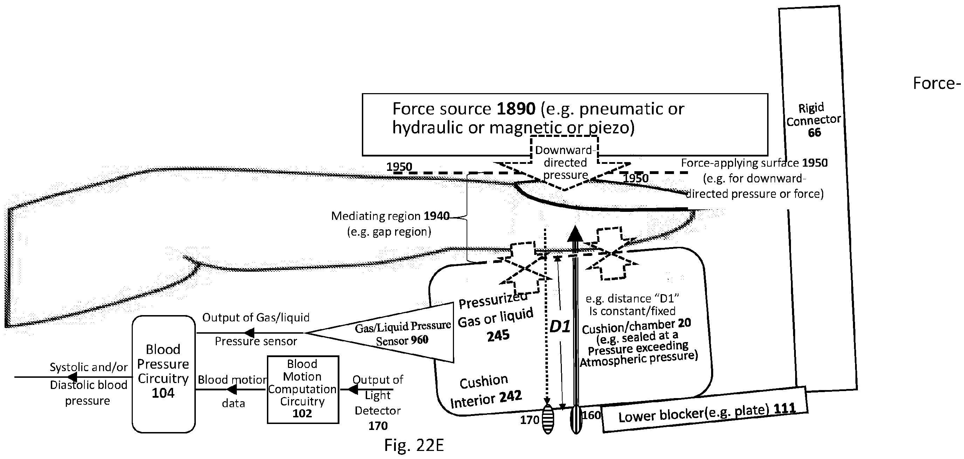

In some embodiments (e.g. see FIG. 22E and 24) the force source **1890** is not based upon pressurized fluid (i.e. not pneumatic or hydraulic). For example, the force source may be a piezo force source or a magnetic force source.

### Theoretical Discussion - Introductory remarks

Embodiments of the invention relate to a method for measuring systolic and/or diastolic blood pressure, based on the measurement of peripheral blood flow. In an example useful for understanding the invention, the method is based on the speckle analysis or dynamic light scattering (DLS) technique. For example, the blood flow dynamics can be characterized in terms of the laser speckle pattern. The blood flow is described by using the laser speckle time domain parameters. This type of analysis enables to reveal different components of the flowing blood including the pulsatile and non-pulsatile.

Practically, the blood pressure Pₚᵤₗₛₑ time variation can be represented as a sum of the P_{DC} (slowly fluctuating in time) and pulsating P_{AC} (fluctuating with the heart rate) components. According to this representation, and taking into account Poiseuille's law, blood flow F also will consist of two parts - the one that fluctuates very slowly (DC) and pulsating component (AC).

The magnitude of AC flow, as it can be represented by DLS technique, follows the shape of the pulse wave. The magnitude of DC flow is defined by the vascular hydrodynamic resistance or impedance. This impedance is dependent also on the activation of the Micsoscopic Venous Valves (MVV). Their MVVs prevent blood reflux in small sized veins and restrict flow from post capillary venules back into the capillary bed

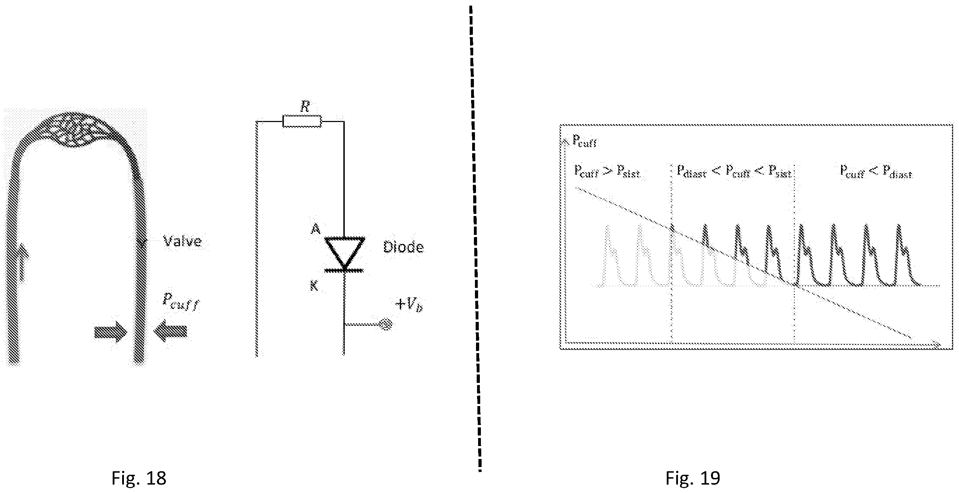

Fig. 18 shows a simplified diagram of a fragment of vascular bed (finger) and its electric counterpart.
These valves are opened only when pressure from the artery side of vascular bed is higher, then at the vein side. Similarly, the diode will be opened only if the voltage at the anode above the cathode voltage, i.e. in the case when blocking voltage is negative. By applying external pressure by means of air cuff, the outflow of blood from the veins is prevented. Veins begin to swell and the pressure in the veins begins to rise. Static condition is achieved when the venous pressure becomes equal to the external pressure (zero transmural pressure). However, until P_{cuff} < P_{diast} blood flow does not cease. After reaching condition P_{diast} < P_{cuff} < P_{syst} the blood flow is interrupting only in those moments, when Pₚᵤₗₛₑ < P_{diast} (Fig. 19).

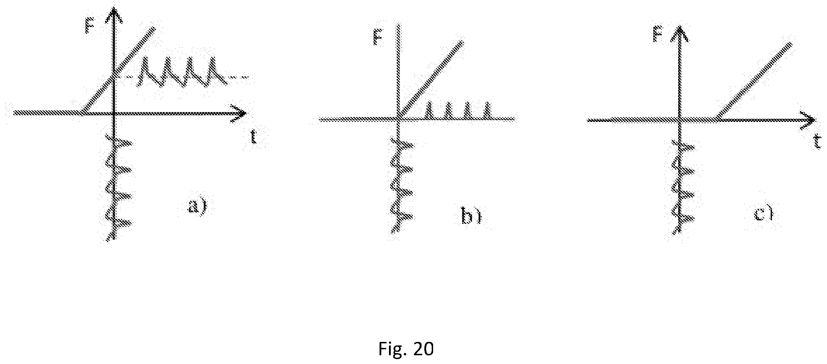

Under these conditions the AC component of the pulse wave is transformed nonlinearly, Fig. 20 shows how the pulse wave transformed by the transfer function F (P) of the venous valves in the 3 regions: a) Pₚᵤₗₛₑ < P_{diast}, b)P_{diast} < P_{cuff} < Pₛᵢₛₜ, and c) P_{cuff} > Pₛᵢₛₜ.

Thus. by changing the external pressure and by measuring the blood flow response, one can find a point of diastolic pressure. If the cuff pressure is increased, the time, during which the valve is open, from the moment when P > P_{diast}, will start to decrease. In terms of the electrical analogy, it may be said that the conduction angle (the part of the cycle during which the diode is conducting) of the diode will start to decrease. While the pressures becomes higher then systolic P > Pₛᵢₛₜ is detected on the base of the flow cessation and loss of pulse signal (conduction angle becomes zero).

If the measurement is made by gradually reducing the pressure (best scenario), we first define the systolic point (when the pulsating flow component appears). With further pressure decrease, we find the diastolic point, basing on the appearance of none-pulsating DC blood stream component.

So, according to some embodiments of the invention, it is possible to quantify the diastolic pressure value based on the appearance of DC blood flow component.

### Theoretical Discussion about why no contact is required

Embodiments of the present invention relate to systems where photodetector(s) receive light reflected from the subject's tissue. For the present disclosure 'tissue' refers to at least skin and optionally at least some additional tissue beneath the skin. The reflected light may include diffusive reflected-light (i.e. scattered light - e.g. scattered off of red blood cells within blood vessels of the subject's tissue) and/or specular reflected light. Not wishing to be bound by theory, it is noted that in contrast to PPG-based systems where a presence a significant specular reflections would destroy any functionality, the presently-disclosed method and system may be more robust. Thus, in some embodiments, at least 10% or at least 25% or at least 50% by power of light (i.e. at the wavelength of the light source) received by each photodetector is specular-reflected light (as opposed to scattered light). The electrical signal generated by each photodetector is processed to compute therefrom the systolic and/or diastolic blood pressures.

Not wishing to be bound by theory, it is noted that specular-reflected light signal is typically characterized by a DC signal or is dominated by low frequencies signal. In contrast, the presently-disclosed DLS-based techniques (in some embodiments thereof) relies on processing the scattered-light-laser speckle optical-response descriptive electrical signal to compute therefrom the BSRD signal, which is then analyzed to compute the systolic and/or diastolic blood pressure. The BSRD is derived primarily from relatively high frequencies within the scattered-light-laser speckle optical-response descriptive electrical signal --- as such, a presence of specular reflection within the optical response signal from the tissue (and within the electrical representation thereof - the scattered-light-laser speckle optical-response descriptive electrical signal) should not significantly reduce the accuracy of the computed hemodynamic information.

In an example useful for understanding the invention, DLS may provide the following feature. This feature is its ability to measure the pulse wave at any location in the body, including the finger root or fingertip. Neither oscillometric nor auscultatory methods provide a significant signal at the finger site. The commonly used optical PPG method can be applied at the finger base or finger tip as well, but it is not related to the Korotkoff sounds, it is not accurate, and is not applicable for the assessment of diastolic pressure.

In the disclosure, unless otherwise stated, adjectives such as "substantially" and "about" that modify a condition or relationship characteristic of a feature or features of an embodiment of the present technology, are to be understood to mean that the condition or characteristic is defined to within tolerances that are acceptable for operation of the embodiment for an application for which it is intended.

While this disclosure has been described in terms of certain embodiments and generally associated methods, alterations and permutations of the embodiments and methods will be apparent to those skilled in the art.

## Claims

1. A system for optically measuring blood pressure of a mammal, the system comprising:
a. a ring assembly **(100)** comprising nested outer **(108)** and inner **(104)** rings disposed around a central axis, the inner ring **(104)** comprising a section that is flexible and optically-transparent, hereafter referred to as FOT section, the outer ring **(108)** comprising a rigid section, the outer and inner rings defining the following three regions:
i. an innermost **(168)** region within the inside of the inner ring **(104) ;**
ii. an annular-shaped mediating region **(188)** outside of the inner ring **(104)** and within the outer ring **(108) ;**
iii. an outermost **(198)** region exterior to the outer ring,
an interior of a gas-sealable inflatable chamber **(120)** being disposed in the mediating region between the FOT section of the inner ring **(104)** and the rigid section of the outer ring **(108)** so that when biological tissue **(159)** of the mammal is disposed in the innermost region, inflation of the chamber **(120)** forces the FOT section of the inner ring to apply an inwardly-directed pressure upon the innermost-region-disposed biological tissue **(159);**
b. an optical blood-motion sensor **(180),** said blood-motion sensor being a laser Doppler sensor, said optical blood-motion sensor **(180)** comprising a laser **(160)** and a light detector **(170)** disposed next to each other, both of the laser **(160)** and the light detector **(170)** being disposed exterior to the inner ring, both of the laser **(160)** and the light detector **(170)** facing inwardly towards the innermost region **(168)** so that when the biological tissue **(159)** is innermost-region-disposed and the FOT section of the inner ring **(104)**applies thereon the inwardly-directed pressure:
i. light emitted by the laser is scattered and reflected by the innermost-region-disposed biological **(159)** tissue after traversing both the interior of the gas-sealable inflatable chamber and the FOT section of the inner ring;
ii. the tissue-scattered and tissue-reflected laser light is received by the light detector after traversing both the FOT section of the inner ring and the interior of the gas-sealable inflatable chamber; and
iii. output of the light detector is electronically processed to compute therefrom a pressure-applied tissue blood motion signal descriptive of blood motion in the biological tissue when subjected to the inwardly-directed pressure; and
c. blood pressure circuitry **(104)** configured to compute a systolic and/or diastolic blood pressure of the mammal by correlating a measurement of a pressure within the inflatable chamber with the pressure-applied tissue blood motion signal computed by the optical blood-movement sensor.

2. The system of claim 1 both of the laser **(160)** and the light detector **(170)** are disposed in the outermost region **198** exterior to the outer ring **108.**

3. A method comprising the steps of:
a. providing the system of any previous claim;
b. disposing biological tissue of the mammal in the innermost region within the inner ring; and
c. employing the system of any previous claim to measure the mammal's blood pressure.

4. The method claim 3 performed so that the light detector receives a mixture of (i) the tissue-scattered and tissue-reflected laser light; and (ii) specular-reflected light.

5. The method of claim 4 wherein at least 10% by power of light received into the photodetector is specular-reflected light.

## Patentansprüche

1. System zur optischen Messung von Blutdruck eines Säugetiers, das System umfassend:
a. eine Ringanordnung **(100),** umfassend einen ineinander geschachtelten äußeren **(108)** und inneren **(104)** Ring, die um eine zentrale Achse angeordnet sind, der innere Ring **(104)** umfassend einen Abschnitt, der flexibel und optisch transparent ist, hierin nachstehend bezeichnet als FOT-Abschnitt,
der äußere Ring **(108)** umfassend einen starren Abschnitt, wobei der äußere und der innere Ring die folgenden drei Bereiche definieren:
i. einen innersten **(168)** Bereich innerhalb der Innenseite des inneren Rings **(104);**
ii. einen ringförmigen Vermittlungsbereich **(188)** außerhalb des inneren Rings **(104)** und innerhalb des äußeren Rings **(108);**
iii. einen äußersten **(198)** Bereich außerhalb des äußeren Rings,
wobei ein Innenraum einer gasabdichtbaren aufblasbaren Kammer **(120),** die in dem Vermittlungsbereich zwischen dem FOT-Abschnitt des inneren Rings **(104)** und dem starren Abschnitt des äußeren Rings **(108)** angeordnet ist, sodass, wenn biologisches Gewebe **(159)** des Säugetiers in dem innersten Bereich angeordnet ist, ein Aufblasen der Kammer **(120)** den FOT-Abschnitt des inneren Rings zwingt, einen nach innen gerichteten Druck auf das in dem innersten Bereich angeordnete biologische Gewebe **(159)** auszuüben;
b. einen optischen Blutbewegungssensor **(180),** wobei der Blutbewegungssensor ein Laser-Doppler-Sensor ist, der optische Blutbewegungssensor **(180)** umfassend einen Laser **(160)** und einen Lichtdetektor **(170),** die nebeneinander angeordnet sind, wobei sowohl der Laser **(160)** als auch der Lichtdetektor **(170)** außerhalb des inneren Rings angeordnet sind, wobei sowohl der Laser **(160)** als auch der Lichtdetektor **(170)** nach innen in Richtung des innersten Bereichs **(168)** weisen, sodass, wenn das biologische Gewebe **(159)** in dem innersten Bereich angeordnet ist und der FOT-Abschnitt des inneren Rings **(104)** darauf den nach innen gerichteten Druck ausübt:
i. Licht, das von dem Laser emittiert wird, von dem in dem innersten Bereich angeordneten biologischen **(159)** Gewebe gestreut und reflektiert wird, nachdem es sowohl den Innenraum der gasabdichtbaren aufblasbaren Kammer als auch den FOT-Abschnitt des inneren Rings durchquert hat;
ii. das von dem Gewebe gestreute und von dem Gewebe reflektierte Laserlicht von dem Lichtdetektor empfangen wird, nachdem es sowohl den FOT-Abschnitt des inneren Rings als auch den Innenraum der gasabdichtbaren aufblasbaren Kammer durchquert hat; und
iii. ein Ausgang des Lichtdetektors elektronisch verarbeitet wird, um daraus ein Signal des Druck beaufschlagtes Gewebebluts zu berechnen, das die Blutbewegung in dem biologischen Gewebe beschreibt, wenn es dem nach innen gerichteten Druck ausgesetzt ist; und
c. Blutdruckschaltungen **(104),** die konfiguriert sind, um einen systolischen und/oder diastolischen Blutdruck des Säugetiers zu berechnen, indem sie eine Messung eines Drucks innerhalb der aufblasbaren Kammer mit dem durch den optischen Blutbewegungssensor berechneten Signal des Druck beaufschlagten Gewebebluts korrelieren.

2. System nach Anspruch 1, wobei sowohl der Laser **(160)** als auch der Lichtdetektor **(170)** in dem äußersten Bereich **198** außerhalb des äußeren Rings **108** angeordnet sind.

3. Verfahren, umfassend die folgenden Schritte:
a. Bereitstellen des Systems nach einem der vorherigen Ansprüche;
b. Anordnen von biologischem Gewebe des Säugetiers in dem innersten Bereich innerhalb des inneren Rings; und
c. Einsetzen des Systems nach einem der vorherigen Ansprüche, um den Blutdruck des Säugetiers zu messen.

4. Verfahren nach Anspruch 3, das ausgeführt wird, sodass der Lichtdetektor ein Gemisch aus (i) dem von dem Gewebe gestreuten und dem von dem Gewebe reflektierten Laserlicht und (ii) dem spiegelreflektierten Licht empfängt.

5. Verfahren nach Anspruch 4, wobei mindestens 10 % der Leistung des in den Photodetektor einfallenden Lichts spiegelreflektiertes Licht ist.

## Revendications

1. Système permettant de mesurer optiquement la pression artérielle d'un mammifère, le système comprenant :
a. un ensemble de bagues **(100)** comprenant des bagues externe **(108)** et interne **(104)** emboîtées disposées autour d'un axe central, la bague interne **(104)** comprenant une section qui est flexible et optiquement transparente, ci-après désignée sous le nom de section FOT,
la bague externe **(108)** comprenant une section rigide, les bagues externe et interne définissant les trois régions suivantes :
i. une région la plus interne **(168)** à l'intérieur de la bague interne **(104)** ;
ii. une région intermédiaire de forme annulaire **(188)** à l'extérieur de la bague interne **(104)** et à l'intérieur de la bague externe **(108)** ;
iii. une région la plus externe **(198)** extérieure à la bague externe,
un intérieur d'une chambre gonflable **(120)** hermétique aux gaz étant disposé dans la région intermédiaire entre la section FOT de la bague interne **(104)** et la section rigide de la bague externe **(108)** de sorte que lorsque le tissu biologique **(159)** du mammifère est disposé dans la région la plus interne, le gonflage de la chambre **(120)** force la section FOT de la bague interne à appliquer une pression dirigée vers l'intérieur sur le tissu biologique **(159)** disposé dans la région la plus interne ;
b. un capteur optique **(180)** de mouvement du sang, ledit capteur de mouvement du sang étant un capteur laser Doppler, ledit capteur optique **(180)** de mouvement du sang comprenant un laser **(160)** et un détecteur de lumière **(170)** disposés l'un à côté de l'autre, le laser **(160)** et le détecteur de lumière **(170)** étant disposés à l'extérieur de la bague interne, le laser **(160)** et le détecteur de lumière **(170)** faisant face vers l'intérieur en direction de la région la plus interne **(168)** de sorte que lorsque le tissu biologique **(159)** est disposé dans la région la plus interne et la section FOT de la bague interne **(104)** applique sur celui-ci la pression dirigée vers l'intérieur :
i. la lumière émise par le laser est diffusée et réfléchie par le tissu biologique **(159)** disposé dans la région la plus interne après avoir traversé à la fois l'intérieur de la chambre gonflable hermétique aux gaz et la section FOT de la bague interne ;
ii. la lumière laser diffusée par le tissu et réfléchie par le tissu est reçue par le détecteur de lumière après avoir traversé à la fois la section FOT de la bague interne et l'intérieur de la chambre gonflable hermétique aux gaz ; et
iii. la sortie du détecteur de lumière est traitée électroniquement pour calculer à partir de celle-ci un signal de mouvement du sang dans le tissu soumis à l'application d'une pression décrivant le mouvement du sang dans le tissu biologique lorsqu'il est soumis à la pression dirigée vers l'intérieur ; et
c. un circuit de pression artérielle **(104)** conçu pour calculer une pression artérielle systolique et/ou diastolique du mammifère en mettant en corrélation la mesure d'une pression à l'intérieur de la chambre gonflable avec le signal de mouvement du sang dans le tissu soumis à l'application d'une tension calculé par le capteur optique de mouvement du sang.

2. Système selon la revendication 1, le laser **(160)** et le détecteur de lumière **(170)** étant disposés dans la région la plus externe **198** à l'extérieur de la bague externe **108.**

3. Procédé comprenant les étapes de :
a. fourniture du système selon l'une quelconque des revendications précédentes ;
b. disposition d'un tissu biologique du mammifère dans la région la plus interne à l'intérieur de la bague interne ; et
c. emploi du système selon l'une quelconque des revendications précédentes pour mesurer la tension artérielle du mammifère.

4. Procédé selon la revendication 3 exécuté de sorte que le détecteur de lumière reçoive un mélange de (i) la lumière laser diffusée par le tissu et réfléchie par le tissu ; et (ii) la lumière réfléchie spéculaire.

5. Procédé selon la revendication 4, au moins 10 % en puissance de la lumière reçue dans le photodétecteur étant de la lumière réfléchie spéculaire.
